(19) Europäisches Patentamt / European Patent Office / Office européen des brevets

(11) **EP 4 628 132 A1**

(12) **DEMANDE DE BREVET EUROPEEN**

(43) Date de publication:
08.10.2025 Bulletin 2025/41

(21) Numéro de dépôt: 25164536.2

(22) Date de dépôt: 18.03.2025

(51) Classification Internationale des Brevets (IPC):
**A61M 16/00** (2006.01)   **A61M 16/10** (2006.01)
**A61M 16/12** (2006.01)   **A61M 16/20** (2006.01)

(52) Classification Coopérative des Brevets (CPC):
**A61M 16/12; A61M 16/0051; A61M 16/024;**
**A61M 16/1005; A61M 16/202; A61M 16/203;**
A61M 16/085; A61M 16/16; A61M 16/22;
A61M 2016/0027; A61M 2016/0033;
A61M 2016/0039; A61M 2016/1025;
A61M 2016/1035; A61M 2202/0208;   (Cont.)

(84) Etats contractants désignés:
AL AT BE BG CH CY CZ DE DK EE ES FI FR GB GR HR HU IE IS IT LI LT LU LV MC ME MK MT NL NO PL PT RO RS SE SI SK SM TR
Etats d'extension désignés:
BA
Etats de validation désignés:
GE KH MA MD TN

(30) Priorité: 04.04.2024 FR 2403481

(71) Demandeur: INOSYSTEMS
92160 Antony (FR)

(72) Inventeurs:
• BLANDIN, Yann
92160 Antony (FR)
• MARCHAL, Frederic
92160 Antony (FR)
• PROUVEZ, Nathan
92160 Antony (FR)
• TIRILLY, Nicolas
92160 Antony (FR)

(74) Mandataire: Air Liquide
L'Air Liquide S.A.
Direction de la Propriété Intellectuelle
75, Quai d'Orsay
75321 Paris Cedex 07 (FR)

(54) **APPAREIL DE FOURNITURE DE NO AVEC MODIFICATION AUTOMATIQUE DES ALARMES LORS DES CHANGEMENTS DE DOSE**

(57) L'invention concerne un appareil de délivrance (1) d'un gaz contenant du NO comprenant des moyens de réglage de dose de NO (50, 51) pour permettre de régler une première dose de NO, et des moyens de pilotage (210) à microprocesseur (211) pour déterminer à partir de la première dose de NO réglée, des premiers seuils d'alarme haut et bas correspondant à des premières teneurs maximale et minimale de NO. Des moyens de modification de dose de NO permettent de modifier la première dose de NO pour obtenir une seconde dose de NO différente de la première dose de NO. Les moyens de pilotage sont configurés pour déterminer automatiquement à partir de la seconde dose de NO, des seconds seuils d'alarme NO haut et bas différents des premiers seuils d'alarme NO haut et bas.

Fig. 5

**(Cont. page suivante)**

(52) Classification Coopérative des Brevets (CPC):
(Cont.)A61M 2202/0275; A61M 2205/18;
A61M 2205/3334; A61M 2205/505;
A61M 2205/581; A61M 2205/583; A61M 2205/75;
A61M 2205/8206

**Description**

L'invention concerne un appareil de délivrance de NO comprenant des moyens de modification automatique des alarmes lors des changements de dose de NO, et une installation de fourniture d'un mélange gazeux à base de NO à un patient, typiquement un mélange NO/azote ($N_2$), comprenant un tel appareil de délivrance de NO et un ventilateur médical délivrant un gaz respiratoire à base d'oxygène ($\geq$ 20% environ).

Le monoxyde d'azote inhalé (NO ou NOi) est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

Pour mettre en œuvre une thérapie par NO inhalé, on utilise une installation de fourniture de gaz, aussi appelée installation d'administration de NO, comprenant un appareil de délivrance de NO et un ventilateur médical, c'est-à-dire un appareil d'assistance respiratoire, alimentant un circuit patient qui comprend généralement un ou plusieurs conduits flexibles reliés fluidiquement à une interface respiratoire, telle une sonde d'intubation trachéale ou analogue, servant à délivrer au patient à traiter, un mélange gazeux final contenant le NO.

**[0004]** Une telle installation de fourniture de gaz est décrite par exemple par EP3821929. Ce type d'installation est utilisé en milieu hospitalier pour administrer le traitement par NO et ainsi soigner les patients ayant besoin d'inhaler du NO pour traiter leur hypertension artérielle pulmonaire. Des installations de ce type sont également décrites par EP3233171, EP3410927, EP3410927, EP4209243, EP4241817, EP4241812 et EP4295882.

Dans une telle installation, l'appareil de délivrance de NO permet d'injecter un flux gazeux à base de NO, typiquement un mélange gazeux NO/azote, dans le circuit patient alimenté par ailleurs en un flux de gaz respiratoire contenant de l'oxygène (au moins environ 20% vol.), tel de l'air ou un mélange oxygène/azote ($O_2/N_2$), fourni par le ventilateur médical, de manière à obtenir un flux combiné, aussi appelé « mélange gazeux final », comprenant le NO à la posologie souhaitée, typiquement moins de 50 ppmv de NO, au moins environ 20% vol. d'oxygène et de l'azote ($N_2$), voire des impuretés inévitables.

Des moyens de contrôle de débit de l'appareil permettent de contrôler ou ajuster le débit de gaz contenant du NO, e.g. de mélange NO/$N_2$, dans le but d'obtenir le mélange gazeux combiné désiré, i.e. le mélange final contenant le NO à la posologie désirée.

En fait, le débit de gaz à base de NO dépend notamment du débit de gaz respiratoire à base d'oxygène, i.e. air ou mélange $N_2/O_2$, provenant du ventilateur médical. Il est donc nécessaire de mesurer en permanence le débit du gaz respiratoire à base d'oxygène grâce à un capteur de débit agencé dans le circuit patient, en amont du site d'injection du mélange à base de NO et d'utiliser ces mesures pour calculer le débit de gaz à base de NO à fournir.

Or, en pratique, on observe des fluctuations plus ou moins importantes de teneur en NO du mélange gazeux combiné, lesquelles résultent de variations du débit de gaz respiratoire à base d'oxygène fourni par le ventilateur médical.

On comprend que de telles variations peuvent être critiques pour le patient si elles sont importantes et conduisent à une teneur en NO (très) éloignée de la posologie désirée, c'est-à-dire si la teneur en NO est excessive ou insuffisante.

Afin de garantir une sécurité accrue pour le patient, l'utilisateur, tel un personnel soignant, peut généralement régler sur l'appareil de délivrance de NO, des valeurs maximale et minimale (i.e. des seuils haut et bas) de teneur en NO acceptables dans le mélange gazeux combiné.

En assurant un monitorage, i.e. un suivi en permanence, de la composition du mélange gazeux combiné obtenu, on peut s'assurer que la proportion de NO qui s'y trouve ne s'écarte pas trop de la posologie désirée. En effet, dans le cas où la teneur en NO mesurée excède l'un des seuils haut ou bas, une alarme sonore et/ou une alarme visuelle peuvent être déclenchée par l'appareil de délivrance de NO afin d'avertir le personnel soignant et lui permettre de prendre des mesures correctrices.

Or, fixer les seuils d'alarme NO haut et bas correspondant aux teneurs (seuils) de NO maximale et minimale acceptables n'est pas chose aisée car ces seuils dépendent étroitement de la dose choisie. Dès lors, le personnel soignant peut commettre des erreurs en les calculant et/ou ensuite en les entrant dans l'appareil de délivrance de NO, et on comprend aisément que toute erreur peut mettre en danger le patient.

De plus, lorsque le personnel soignant, tel un médecin, décide de changer la dose de NO afin d'adapter le traitement du patient, c'est-à-dire d'augmenter ou diminuer la dose de NO dans le mélange gazeux combiné envoyé au patient, il doit penser à aller modifier aussi les seuils d'alarme NO haut et bas ayant été fixés pour la dose précédente afin d'en choisir de nouveaux correspondant à la nouvelle dose de NO choisies par le médecin.

Là encore, outre le fait que le médecin peut oublier d'adapter les seuils d'alarme à la nouvelle dose de NO, des erreurs peuvent se produire lors de la saisie des seuils avec les mêmes conséquences négatives pour le patient que susmentionnées.

Dans tous les cas, des seuils d'alarme mal choisis ou mal réglés engendrent inévitablement des déclenchements intempestifs d'alarme qui conduisent notamment à des nuisances sonores et à une fatigue accrue pour le personnel soignant qui doit intervenir sans cesse pour les inactiver en les détournant alors de tâches plus critiques à opérer. Ceci qui nuit alors au traitement du patient, notamment à sa sécurité.

US2022106189 propose un système de production, à partir de $NO_2$, d'un mélange gazeux contenant du NO susceptible d'être administré à un patient, lequel système inclut un analyseur de gaz. Une fois une dose fixée, le système calcule des seuils d'alarme haut et bas. Le seul exemple proposé repose sur un calcul de seuils de 7 ppm et 13 ppm, pour une dose de NO égale à 10 ppm, soit un écart de +/- 30%. Toutefois, rien n'est précisé quant au recalcul des seuils en cas de modification ultérieure de la dose de NO, en particulier pour les doses inférieures à 10 ppm.

US11833309 propose également un dispositif générateur de NO, dans lequel les seuils d'alarme sont calculés ou recalculés automatiquement lors de la fixation ou d'un changement de dose de NO. Il y est précisé qu'on peut soit calculer les seuils en tant que pourcentages autour de la valeur de NO souhaitée, soit utiliser une table de correspondances préétablie. Toutefois, aucune information quant au pourcentage à appliquer n'y est donné et la question des doses de moins de 10 ppm n'y est pas adressée.

Par ailleurs, US2013/118486 et US2013/192595 enseignent un appareil d'administration de NO avec surveillance de la teneur de NO fournie où une alarme est déclenchée lorsqu'un écart est déterminé. Si des valeurs d'écart comprises entre +/-1% et +/-100% sont citées, il y est précisé que l'écart est préférentiellement d'au moins 25%. La question des doses de moins de 10 ppm n'y est pas adressée, en particulier en cas de re-calcul des seuils suite à un changement de la dose initiale.

Un problème est dès lors d'améliorer la sécurité du patient, en particulier de pouvoir éviter ou au moins minimiser les risques susmentionnés et/ou les déclenchements intempestifs d'alarme, tout en assurant un traitement efficace du patient, en proposant un appareil de délivrance de NO amélioré, c'est-à-dire plus sécuritaire quant à la détermination des seuils d'alarme NO, typiquement en cas de changement de dose de NO, en particulier pour les doses de NO faibles, c'est-à-dire de moins de 10 ppmv.

Une solution concerne un appareil de délivrance, i.e. de fourniture, d'un gaz contenant du NO, tel un mélange gazeux $NO/N_2$, comprenant des moyens de réglage de dose de NO configurés pour permettre à un utilisateur de régler, i.e. fixer, sélectionner ou analogue, une première dose de NO ($D_{NO1}$) comprise entre 1 et 80 ppmv, et des moyens de pilotage à microprocesseur configurés pour déterminer à partir de ladite première dose de NO réglée ($D_{NO1}$), des premiers seuils d'alarme haut et bas correspondant à une première teneur maximale de NO ($T_{max1}$) et à une première teneur minimale de NO ($T_{min1}$), avec : $T_{max1} > D_{NO1} > T_{min1}$.

Par ailleurs, l'appareil de délivrance de l'invention comprend en outre des moyens de modification de dose de NO configurées pour permettre à un utilisateur de modifier ou ajuster la première dose de NO ($D_{NO1}$) pour obtenir ou fixer une seconde dose de NO ($D_{NO2}$) différente de la première dose de NO ($D_{NO1}$), et les moyens de pilotage sont configurés pour déterminer, i.e. calculer, modifier, ajuster ou analogue, automatiquement à partir de ladite seconde dose de NO ($D_{NO2}$), des seconds seuils d'alarme NO haut et bas différents desdits premiers seuils d'alarme NO haut et bas, correspondant à une seconde teneur maximale de NO ($T_{max2}$) et à une seconde teneur minimale de NO ($T_{min2}$), avec : $T_{max2} > D_{NO2} > T_{min2}$.

De plus, les moyens de pilotage sont configurés pour que la seconde teneur maximale de NO ($T_{max2}$) et la seconde teneur minimale de NO ($T_{min2}$) automatiquement déterminées soient telles que :

$$T_{max2} \leq 1{,}20 \cdot D_{NO2} \quad \text{et} \quad 0{,}80 \cdot D_{NO2} \leq T_{min2}$$

avec comme condition supplémentaire que, lorsque la seconde dose de NO ($D_{NO2}$) est inférieure à 10 ppmv, lesdites secondes teneurs maximale et minimale de NO ($T_{max2}$, $T_{min2}$) sont telles que : $T_{max2} - D_{NO2} \geq 2$ ppmv et $D_{NO2} - T_{min2} \geq 2$ ppmv.

De préférence, secondes teneurs maximale et minimale de NO ($T_{max2}$, $T_{min2}$) sont telles que : $T_{max2} - D_{NO2} = 2$ ppmv et $D_{NO2} - T_{min2} = 2$ ppmv.

Autrement dit, au lesdites début du traitement d'un patient, c'est-à-dire juste avant le démarrage de fourniture de NO au patient, les moyens de pilotage de l'appareil déterminent, i.e. calculent, automatiquement les valeurs des premiers seuils d'alarme NO haut et bas, c'est-à-dire lesdites premières teneurs maximale et minimale de NO ($T_{min1}$, $T_{max1}$), à partir de la valeur de la première dose de NO ($D_{NO1}$) ou posologie réglée par l'utilisateur, i.e. le médecin ou analogue, en ajoutant ou, à l'inverse, retirant de 10% à 20% à la valeur de dose de NO ($D_{NO}$) réglée, de préférence de l'ordre de 20% qui correspond à une tolérance acceptable et ce, pour une valeur de première dose de NO ($D_{NO1}$) généralement comprise entre 1 et 80 ppmv.

Ensuite, si l'utilisateur, typiquement un médecin, décide de changer la posologie, c'est-à-dire de modifier la dose de NO pour la faire passer de la première dose de NO ($D_{NO1}$) à une seconde dose de NO ($D_{NO2}$), qui est différente de la première dose de NO ($D_{NO1}$), alors l'appareil, typiquement son microprocesseur, va calculer automatiquement, à partir de cette seconde dose de NO ($D_{NO2}$), des seconds seuils d'alarme NO haut et bas différents desdits premiers seuils d'alarme NO haut et bas, correspondant des secondes teneurs maximale de NO ($T_{max2}$) minimale de NO ($T_{min2}$), et avec : $T_{max2} > D_{NO2} > T_{min2}$.

Le calcul se fait comme pour les premiers seuils d'alarme, c'est-à-dire en appliquant la même tolérance qui est inférieure ou égale à 20%, par exemple 20%.

Le fait que les seuils d'alarme NO soient recalculés automatiquement et immédiatement, c'est-à-dire de manière instantanée, par l'appareil de délivrance de NO dès que l'utilisateur a fixé la seconde dose de NO ($D_{NO2}$) désirée, c'est-à-dire la seconde posologie souhaitée, est un avantage indéniable en termes de sécurité car les erreurs de calcul ou d'entrée des valeurs dans l'appareil ou la possibilité que le personnel soignant oubli de recalculer ces valeurs n'existent plus.

De plus, lorsque la seconde dose de NO ($D_{NO2}$) a une valeur faible, c'est-à-dire lorsqu'elle est inférieure à 10 ppmv, l'appareil va calculer automatiquement, à partir de cette seconde dose de NO ($D_{NO2}$), des seconds seuils d'alarme NO haut et bas particuliers s'écartant d'au moins 2 ppmv de la nouvelle dose de NO ($D_{NO2}$) ayant été réglée ou sélectionnée par l'utilisateur, i.e. médecin ou analogue, de préférence un écart de 2 ppmv. Ceci évite des déclenchements d'alarmes inopinées, i.e. de fausses alarmes et la sécurité du patient s'en trouve améliorée, ainsi que l'efficacité de traitement, en particulier pour les patients pédiatriques.

Selon le mode de réalisation considéré, l'appareil de délivrance de l'invention peut comprendre l'une ou plusieurs des caractéristiques suivantes :

- les moyens de pilotage sont configurés pour calculer (i.e. déterminer) automatiquement, à partir de la première dose de NO ($D_{NO1}$) réglée, un premier seuil d'alarme NO haut correspondant à une première teneur maximale de NO ($T_{max1}$), telle que : $1,1 . D_{NO1} \leq T_{max1} \leq 1.2 . D_{NO1}$, c'est-à-dire que la première teneur maximale de NO ($T_{max1}$) est supérieure de 10% à 20% à la première valeur de dose de NO ($D_{NO1}$) réglée.
- les moyens de pilotage sont configurés pour calculer (i.e. déterminer) automatiquement, à partir de la première dose de NO ($D_{NO1}$) réglée, un premier seuil d'alarme NO bas correspondant à une première teneur minimale de NO ($T_{min1}$), telle que : $0.8 . D_{NO1} \leq T_{min1} \leq 0,9 . D_{NO1}$, c'est-à-dire que la première teneur minimale de NO ($T_{min1}$) est inférieure de 10% à 20% à la première valeur de dose de NO ($D_{NO1}$) réglée.
- les moyens de pilotage sont configurés pour calculer (i.e. déterminer), à partir de la première dose de NO ($D_{NO1}$) réglée, un premier seuil d'alarme NO haut correspondant à une première teneur maximale de NO ($T_{max1}$), telle que : $T_{max1} \leq 1,20 . D_{NO1}$, en particulier telle que : $T_{max1} = 1,20 . D_{NO1}$.
- les moyens de pilotage sont configurés pour calculer (i.e. déterminer), à partir de la première dose de NO ($D_{NO1}$) réglée, un premier seuil d'alarme NO bas correspondant à une première teneur minimale de NO ($T_{min1}$), telle que : $0,80 . D_{NO1} \leq T_{min1}$, en particulier telle que : $0,80 . D_{NO1} = T_{min1}$ .
- selon un mode de réalisation préféré, les moyens de pilotage sont configurés pour calculer (i.e. déterminer), à partir de la première dose de NO ($D_{NO1}$) réglée, des premiers seuils d'alarme NO haut et bas s'écartant au maximum de 20% de la première dose de NO ($D_{NO1}$), c'est-à-dire, en d'autres termes :

    o un premier seuil d'alarme NO haut correspondant à une première teneur maximale de NO ($T_{max1}$), telle que : $T_{max1} \leq 1,20 . D_{NO1}$, de préférence :

$$T_{max1} = 1,20 . D_{NO1},$$

    et
    o un premier seuil d'alarme NO bas correspondant à une première teneur minimale de NO ($T_{min1}$), telle que : $0,80 . D_{NO1} \leq T_{min1}$, de préférence :

$$0,80 . D_{NO1} = T_{min1}.$$

- avantageusement, les moyens de pilotage sont configurés pour calculer une première teneur maximale de NO ($T_{max1}$) telle que : $T_{max1} - D_{NO1} \geq 2$ ppmv , c'est-à-dire que l'écart entre la première dose de NO ($D_{NO1}$) réglée et la première teneur maximale de NO ($T_{max1}$) doit être au minimum de 2 ppmv, en particulier lorsque la première dose de NO ($D_{NO1}$) est inférieure à 10 ppmv. Par exemple, pour une première dose de NO ($D_{NO1}$) de 10 ppmv, la première teneur maximale de NO ($T_{max1}$) doit être fixée à au moins 12 ppmv, i.e. 12 ppmv ou plus, alors que par exemple pour une dose de 5 ppmv, la première teneur maximale de NO ($T_{max1}$) doit être fixée à au moins 7 ppmv (i.e. $\geq 7$ ppm).
- les moyens de pilotage sont en outre configurés pour calculer une première teneur minimale de NO ($T_{min1}$) telles que : $D_{NO1} - T_{min1} \geq 2$ ppmv , c'est-à-dire que, là aussi, l'écart entre la première dose de NO ($D_{NO1}$) réglée et la première teneur minimale de NO ($T_{min1}$) doit être au minimum de 2 ppmv, en particulier lorsque la première dose de NO ($D_{NO1}$) est inférieure à 10 ppmv. Par exemple, pour une première dose de NO ($D_{NO1}$) de 10 ppmv, la première teneur minimale de NO ($T_{min1}$) doit être fixée à au plus 8 ppmv, i.e. 8 ppm ou moins, alors que par exemple pour une dose de 5 ppmv, la première teneur minimale de NO ($T_{min1}$) doit être fixée à au plus 3 ppmv (i.e. $\leq 3$ ppm).
- il comprend un afficheur graphique, c'est-à-dire un écran d'affichage ou analogue.
- l'afficheur graphique est configuré pour afficher au moins ladite première dose de NO réglée ($D_{NO1}$) et/ou lesdites premières teneurs maximale et minimale de NO ($T_{min1}$, $T_{max1}$) correspondant auxdits premiers seuils d'alarme haut et bas.

- l'afficheur graphique comprend un écran tactile (i.e. à dalle tactile) ou tout autre dispositif d'affichage équivalent.
- les moyens de pilotage sont configurés pour commander un affichage, sur l'afficheur graphique, de la première teneur maximale de NO ($T_{max1}$) correspondant au premier seuil d'alarme NO haut.
- les moyens de pilotage sont configurés pour commander un affichage sur l'afficheur graphique de la première teneur minimale de NO ($T_{min1}$) correspondant au premier seuil d'alarme NO bas.
- la première dose de NO ($D_{NO1}$) est comprise entre 1 et 60 ppmv.
- les moyens de pilotage sont par ailleurs configurés pour déterminer, e.g. calculer, automatiquement, à partir de la seconde dose de NO ($D_{NO2}$) réglée, un second seuil d'alarme NO haut correspondant à une seconde teneur maximale de NO ($T_{max2}$) et un second seuil d'alarme NO bas correspondant à une seconde teneur minimale de NO ($T_{min2}$), ladite détermination étant opérée de la même manière que celle du premier seuil d'alarme NO haut correspondant à la première teneur maximale de NO ($T_{max1}$) et du premier seuil d'alarme NO bas correspondant à la première teneur minimale de NO ($T_{min1}$).
- les moyens de pilotage sont par ailleurs configurés pour calculer (i.e. déterminer) automatiquement, à partir de la seconde dose de NO ($D_{NO2}$) réglée, un second seuil d'alarme NO haut correspondant à une seconde teneur maximale de NO ($T_{max2}$), telle que : $1,1 . D_{NO2} \leq T_{max2} \leq 1,2 . D_{NO2}$, c'est-à-dire que la seconde teneur maximale de NO ($T_{max2}$) est supérieure de 10% à 20% à la seconde valeur de dose de NO ($D_{NO2}$) réglée.
- les moyens de pilotage sont configurés pour calculer automatiquement, à partir de la seconde dose de NO ($D_{NO2}$) réglée, un second seuil d'alarme NO bas correspondant à une seconde teneur minimale de NO ($T_{min2}$), telle que : $0,7 . D_{NO2} \leq T_{min2} \leq 0,8 . D_{NO2}$, c'est-à-dire que la seconde teneur minimale de NO ($T_{min2}$) est inférieure de 10% à 20% à la seconde valeur de dose de NO ($D_{NO2}$) réglée.
- les moyens de pilotage sont configurés pour calculer, à partir de la seconde dose de NO ($D_{NO2}$) réglée, un second seuil d'alarme NO haut correspondant à une seconde teneur maximale de NO ($T_{max2}$), telle que : $T_{max2} \leq 1,20 . D_{NO2}$, en particulier telle que : $T_{max2} = 1,20 . D_{NO2}$.
- les moyens de pilotage sont configurés pour calculer, à partir de la seconde dose de NO ($D_{NO2}$) réglée, un second seuil d'alarme NO bas correspondant à une seconde teneur minimale de NO ($T_{min2}$), telle que : $0,80 . D_{NO2} \leq T_{min2}$, en particulier telle que : $0,80 . D_{NO2} = T_{min2}$.
- là encore, avantageusement, les moyens de pilotage sont configurés pour calculer une seconde teneur maximale de NO ($T_{max2}$) telle que : $T_{max2} - D_{NO2} \geq 2$ ppmv, c'est-à-dire que l'écart entre la seconde dose de NO ($D_{NO2}$) réglée et la seconde teneur maximale de NO ($T_{max2}$) doit être au minimum de 2 ppmv, en particulier lorsque la seconde dose de NO ($D_{NO2}$) est inférieure ou égale à 10 ppmv.
- par analogie, les moyens de pilotage sont en outre configurés pour calculer une seconde teneur minimale de NO ($T_{min2}$) telles que : $D_{NO2} - T_{min2} \geq 2$ ppmv, c'est-à-dire que, là aussi, l'écart entre la seconde dose de NO ($D_{NO2}$) réglée et la seconde teneur minimale de NO ($T_{min2}$) doit être au minimum de 2 ppmv, en particulier lorsque la seconde dose de NO ($D_{NO2}$) est inférieure ou égale à 10 ppmv.
- l'afficheur graphique est en outre configuré pour afficher au moins ladite seconde dose de NO ($D_{NO2}$) et/ou lesdites secondes teneurs maximale et minimale de NO ($T_{min2}$, $T_{max2}$) correspondant auxdits seconds seuils d'alarme NO haut et bas ayant été déterminés.
- les moyens de pilotage sont configurés pour commander un affichage sur l'afficheur graphique de la seconde teneur maximale de NO ($T_{max2}$) correspondant au second seuil d'alarme NO haut et de la seconde teneur minimale de NO ($T_{min2}$) correspondant au second seuil d'alarme NO bas.
- la seconde dose de NO ($D_{NO2}$) est comprise entre 1 et 80 ppmv.
- la seconde dose de NO ($D_{NO2}$) est comprise entre 1 et 75 ppmv, de préférence entre 1 et 55 ppmv.
- la seconde dose de NO ($D_{NO2}$) est inférieure à la première dose de NO ($D_{NO1}$) ou, alternativement, la seconde dose de NO ($D_{NO2}$) est supérieure à la première dose de NO ($D_{NO1}$).
- l'écart (en valeur absolue) entre la première dose de NO ($D_{NO1}$) et la seconde dose de NO ($D_{NO2}$) est d'au moins 2 ppmv, de préférence d'au moins 3 ppmv, de préférence d'au moins 4 ppmv, par exemple d'au moins 5 ppmv.
- l'afficheur graphique est configuré pour afficher simultanément la seconde dose de NO ($D_{NO2}$) et les secondes teneurs maximale et minimale de NO ($T_{min2}$, $T_{max2}$) correspondant auxdits seconds seuils d'alarme NO haut et bas ayant été déterminés.
- les moyens de pilotage sont configurés pour déterminer les secondes teneurs minimale et maximale de NO ($T_{min2}$, $T_{max2}$) telles que, lorsque : $D_{NO2} < D_{NO1}$ alors : $T_{max2} < T_{max1}$ et $T_{min2} < T_{min1}$.
- les moyens de pilotage sont configurés pour déterminer les secondes teneurs minimale et maximale de NO ($T_{min2}$, $T_{max2}$) telles que, lorsque : $D_{NO2} > D_{NO1}$ alors : $T_{max2} > T_{max1}$ et $T_{min2} > T_{min1}$.
- il comprend en outre des moyens de mémorisation pour mémoriser la première dose de NO ($D_{NO1}$) et/ou la seconde dose de NO ($D_{NO2}$).
- les moyens de mémorisation permettent de mémoriser la première teneur maximale de NO ($T_{max1}$) et/ou la première teneur minimale de NO ($T_{min1}$).
- les moyens de mémorisation permettent de mémoriser la seconde teneur maximale de NO ($T_{max2}$) et/ou la seconde teneur minimale de NO ($T_{min2}$).

- il comprend un circuit de gaz interne pour acheminer un flux de gaz contenant du NO, c'est-à-dire des passages de gaz, conduits de gaz ou analogues.
- le circuit de gaz interne comprend des moyens de contrôle de débit configurés pour contrôler le flux de gaz contenant du NO au sein dudit circuit de gaz.
- les moyens de pilotage sont configurés pour piloter (au moins) les moyens de contrôle de débit pour contrôler la fourniture de gaz contenant du NO en fonction d'au moins ladite première dose de NO réglée ($D_{NO1}$) ou, selon le cas, de la seconde dose de NO ($D_{NO2}$), c'est-à-dire quand la dose de NO a été modifiée avec passage de la première dose de NO ($D_{NO1}$) à la seconde dose de NO ($D_{NO2}$).
- le circuit de gaz interne de l'appareil véhiculant le gaz contenant le NO, typiquement un mélange gazeux $NO/N_2$, est raccordé fluidiquement à un circuit respiratoire véhiculant un gaz respiratoire contenant de l'oxygène, tel de l'air ou un mélange gazeux $O_2/N_2$ (>20% $O_2$), pour y injecter le gaz contenant le NO et obtenir un mélange gazeux combiné contenant du NO et de l'oxygène, c'est-à-dire le mélange final à administrer au patient.
- il comprend des moyens de mesure de concentration de NO pour déterminer la teneur en NO dans le mélange gazeux combiné contenant du NO et de l'oxygène, tel un capteur de NO, par exemple à cellule électrochimique.
- les moyens de mesure de concentration de NO sont agencés de manière à déterminer la teneur en NO dans le mélange gazeux combiné au sein du circuit de gaz interne.
- il comprend des moyens d'alarme, typiquement une alarme sonore et/ou visuelle.
- les moyens de pilotage sont configurés pour agir sur des moyens d'alarme pour déclencher une alarme, en particulier une alarme sonore et/ou visuelle.
- les moyens d'alarme peuvent comprendre un dispositif de type buzzer, et/ou un haut-parleur, ou tout autre dispositif permettant d'émettre un signal sonore audible par l'oreille humaine, i.e. par le personnel soignant.
- les moyens d'alarme peuvent comprendre un dispositif lumineux, par exemple comprenant une ou des diodes (LED) ou analogue.
- les moyens d'alarme peuvent comprendre un affichage d'un message d'alerte ou analogue sur l'afficheur graphique.
- les moyens de pilotage sont configurés pour déclencher une alarme, i.e. pour activer des moyens d'alarme, lorsque la teneur en NO (i.e. une teneur instantanée) dans le mélange gazeux combiné est supérieure ou égale à la première ou, selon le cas, à la seconde teneur maximale de NO ($T_{max1}$, $T_{max2}$) ou inférieure ou égale à la première ou, selon le cas, à la seconde teneur minimale de NO ($T_{min1}$, $T_{min2}$).
- les moyens de pilotage déterminent la teneur en NO (i.e. une teneur instantanée) dans le mélange gazeux combiné à partir de mesures fournies par les moyens de mesure de concentration de NO.
- les moyens de pilotage sont configurés pour commander un affichage sur l'afficheur graphique de la teneur en NO dans le mélange gazeux combiné ayant été déterminée par les moyens de mesure de concentration de NO.
- l'afficheur est configuré pour afficher un message d'alarme, en cas de déclenchement d'une alarme.
- les moyens de réglage de dose et/ou les moyens de modification de dose de NO sont configurés pour permettre à l'utilisateur de modifier, régler, ajuster ou analogue la dose de NO ($D_{NO1}$, $D_{NO2}$) et/ou les seuils d'alarme NO haut et/ou bas ($T_{max1}$, $T_{max2}$, $T_{min1}$, $T_{min2}$).
- les moyens de réglage de dose et/ou les moyens de modification de dose de NO comprennent une ou des touches ou analogues, en particulier des touches virtuelles affichées par l'écran d'affichage.
- il est alimenté en un gaz contenant du NO en une proportion initiale donnée, typiquement un mélange $NO/N_2$.
- il est alimenté en un gaz contenant une proportion initiale de NO comprise entre 100 et 2000 ppmv, de péférence entre 100 et 1500 ppmv, typiquement entre 200 et 1000 ppmv.
- il est alimenté en un mélange gazeux formé d'azote et de NO.
- les moyens de réglage ou de modification de dose de NO sont configurés pour permettre à l'utilisateur de régler, ajuster ou modifier une dose de NO, i.e. consigne de teneur en NO, correspondant à la proportion de NO souhaitée dans le mélange gazeux combiné, c'est-à-dire après mélange du mélange $NO/N_2$ avec le flux de gaz respiratoire à base d'oxygène, i.e. une posologie en NO.
- les moyens de réglage ou de modification de dose de NO font partie d'une IHM (interface homme-machine) ou IGU (interface graphique utilisateur).
- l'IHM comprend l'afficheur graphique.
- préférentiellement, les moyens de réglage de dose ou de modification de NO comprennent une ou des touches tactiles, actionnables par l'utilisateur, s'affichant sur un afficheur graphique de type écran digital à dalle tactile, c'est-à-dire des touches virtuelles.
- les moyens de réglage de dose et/ou les moyens de modification de dose de NO comprennent une touche « + » permettant d'incrémenter une valeur de dose de NO et/ou une touche « - » permettant de décrémenter une valeur de dose de NO. Typiquement, l'incrémentation ou la décrémentation se fait de +/- 1 ppmv.
- les moyens de réglage de dose et/ou les moyens de modification de dose de NO comprennent en outre une touche de validation pour valider/confirmer une valeur de dose de NO ayant été fixée, de préférence une touche de validation virtuelle affichée par l'écran d'affichage.

- alternativement, les moyens de réglage ou de modification de dose de NO comprennent un organe de sélection mécanique, tel un (des) boutons ou touches à actionnement digital, tel un bouton-poussoir ou analogue, ou un bouton rotatif ou analogue, servant à sélectionner, choisir, ajuster, fixer, régler, valider ou analogue, une valeur de dose de NO, telle qu'une valeur de départ ou une nouvelle valeur en cas de modification de dose.
- le calcul des seuils d'alarme par les moyens de pilotage, en particulier en cas de modification de la dose de NO à administrer, i.e. la seconde dose de NO ($D_{NO2}$), se fait de manière automatique et instantanée, c'est-à-dire en temps réel et en réponse à la modification de la dose de NO à administrer, typiquement après validation de la nouvelle dose par l'opérateur.
- l'afficheur graphique est du type à affichage en couleurs.
- les moyens de réglage ou de modification de dose de NO sont configurés pour permettre à l'utilisateur de régler une dose de NO, i.e. une consigne de teneur en NO.
- les doses ou consignes de teneur en NO ($D_{NO1}$, $D_{NO2}$) sont comprises entre 1 et 70 ppmv, de préférence entre 1 et 60 ppmv, avantageusement entre 1 et 50 ppmv, typiquement entre 5 et 40 ppmv.
- les moyens de mémorisation comprennent une mémoire informatique, telle une mémoire flash, une RAM ou analogue.
- les moyens de contrôle de débit sont agencés sur le circuit de gaz interne.
- les moyens de contrôle de débit comprennent des moyens à vannes, telles des électrovannes ou analogues.
- les moyens de pilotage sont configurés pour contrôler les moyens de contrôle de débit pour autoriser ou empêcher le passage du flux de NO/$N_2$ dans le circuit de gaz interne.
- les moyens de contrôle de débit comprennent une (ou des) électrovanne proportionnelle et/ou une (ou des) électrovanne tout ou rien.
- les moyens de contrôle de débit principaux comprennent un contrôleur de débit massique (Mass Flow Controller).
- le contrôleur de débit massique (Mass Flow Controller) ou MFC comprend au moins une électrovanne proportionnelle pilotée par les moyens de pilotage et un capteur de débit principal.
- les moyens de pilotage à microprocesseur comprennent un (micro)contrôleur ou analogue.
- les moyens de pilotage à microprocesseur comprennent un (ou des) (micro)processeur agencé sur une (ou des) carte électronique.
- les moyens de pilotage comprennent un (ou des) (micro)processeur mettant en œuvre un ou des algorithmes, notamment un (ou des) algorithme de pilotage des moyens de contrôle de débit, un (ou des) algorithme de traitement des mesures de débit de gaz respiratoire ou de mesures de concentration en NO, $NO_2$ et/ou en $O_2$....
- les moyens de mémorisation sont agencés sur la carte électronique.

L'invention porte aussi sur une installation de fourniture d'un gaz contenant du NO comprenant l'appareil de délivrance de NO selon l'invention alimenté en un mélange gazeux NO/$N_2$ par une source de mélange NO/$N_2$, et un ventilateur médical configuré pour fournir un flux de gaz respiratoire contenant de l'$O_2$, typiquement à un circuit respiratoire véhiculant le flux de gaz respiratoire sortant du ventilateur médical.

Selon le mode de réalisation considéré, l'installation de fourniture d'un gaz contenant du NO de l'invention peut comprendre l'une ou plusieurs des caractéristiques suivantes :

- le ventilateur médical est configuré pour fournir un flux de gaz respiratoire contenant au moins 20%vol. environ d'$O_2$, typiquement un mélange gazeux NO/$N_2$ ou de l'air.
- l'appareil de délivrance de NO et le ventilateur médical sont raccordés fluidiquement au circuit respiratoire pour lui fournir des flux gazeux.
- un capteur de débit est agencé dans le circuit respiratoire entre le ventilateur médical et le dispositif d'injection.
- un dispositif d'injection est agencé dans le circuit respiratoire, en aval du capteur de débit.
- le dispositif d'injection est configuré pour permettre d'injecter le gaz contenant du NO provenant de l'appareil de délivrance de NO dans le flux de gaz respiratoire contenant de l'$O_2$ provenant du ventilateur médical pour obtenir le mélange gazeux combiné à fournir au patient en ayant besoin.
- le dispositif d'injection est configuré pour opérer un mélange du gaz contenant du NO provenant de l'appareil de délivrance de NO avec le flux de gaz respiratoire contenant de l'$O_2$ acheminé par le circuit respiratoire, et obtenir un mélange gazeux combiné contenant du NO et de l'oxygène, voire d'autres composés comme de l'azote ou des impuretés inévitables.
- le dispositif d'injection comprend une première entrée de gaz alimentée en flux de gaz respiratoire contenant de l'$O_2$, i.e. provenant du ventilateur médical.
- le dispositif d'injection comprend en outre une seconde entrée de gaz alimentée en gaz contenant du NO provenant de l'appareil de délivrance de NO.
- le dispositif d'injection comprend en outre une sortie de gaz fournissant le mélange gazeux combiné contenant du NO et de l'oxygène, obtenu par mélange, au sein du dispositif d'injection, du gaz contenant du NO (e.g. mélange NO/N$_2$) avec le flux de gaz respiratoire contenant de l'O$_2$ (e.g. air ou mélange O$_2$/N$_2$).

- le ventilateur médical délivre de l'air ou un mélange oxygène/azote, i.e. en tant que gaz respiratoire contenant au moins 20% vol. environ d'oxygène, de préférence au moins 21% vol. environ d'oxygène.
- le ventilateur médical comprend une soufflante motorisée (i.e. turbine, compresseur ou analogue) délivrant le gaz respiratoire, typiquement de l'air ou un mélange oxygène/azote ou, selon un autre mode de réalisation, un circuit de gaz interne comprenant une ou des vannes proportionnelles pour acheminer le gaz et contrôler sa fourniture, notamment son débit. Un tel ventilateur est généralement alimenté en gaz respiratoire par une (ou des) prise murale alimentée en gaz par un réseau de canalisations d'un établissement ou bâtiment hospitalier, typiquement de l'air ou un mélange oxygène/azote.
- le ventilateur médical comprend des moyens ou un dispositif de commande, telle une (ou des) carte de commande électronique. De préférence, les moyens de commande du ventilateur médical pilotent ou commandent la soufflante motorisée ou, selon le cas, les valves proportionnelles du ventilateur médical.
- le ventilateur médical est de type HFO ou comprend une fonction de HFO, c'est-à-dire qu'il est apte à produire des oscillations à haute fréquence.
- la (les) source de NO alimentant l'appareil de délivrance de NO contient un mélange gazeux NO/N$_2$ contenant entre 100 et 2000 ppmv de NO, le reste étant de l'azote (N$_2$), de préférence entre 100 et 1000 ppmv de NO, conditionné à une pression comprise entre 10 et 250 bar abs, typiquement à plus de 100 bar abs (avant début de soutirage).
- la source de NO est ou comprend une (ou des) bouteille de gaz ayant une contenance comprise entre 0,5 et 50 L (équivalent en eau).
- la (les) bouteille de gaz comprend un corps cylindrique en acier ou en alliage d'aluminium et est équipée d'un robinet simple (sans détendeur) ou à détendeur intégré ou RDI, de préférence un RDI, protégé par un capotage de protection, par exemple en métal ou polymère.
- le circuit respiratoire (i.e. circuit patient) comprend une branche inspiratoire et une branche expiratoire, typiquement des conduites flexibles formant la branche inspiratoire et la branche expiratoire, par exemple des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- l'interface respiratoire patient comprend une sonde d'intubation trachéale ou masque respiratoire, ou autre.
- le circuit respiratoire, en particulier la branche inspiratoire, peut comprendre un humidificateur de gaz.
- l'humidificateur de gaz est agencé en aval du dispositif d'injection, par exemple un module d'injection de NO, de manière à pouvoir humidifier le gaz avant son administration par inhalation au patient.
- le ventilateur et l'appareil de délivrance de NO sont alimentés électriquement par une ou des sources de courant électrique, typiquement le secteur (110/220V) et/ou une ou des batteries rechargeables.

Selon un autre aspect, l'invention concerne aussi une méthode de traitement thérapeutique d'une personne, i.e. un patient humain (i.e. adulte, enfant, adolescent ou nouveau-né), souffrant d'hypertension pulmonaire et/ou d'hypoxie, engendrant des vasoconstrictions pulmonaires ou analogues, comprenant une administration par inhalation à la personne en ayant besoin, d'un mélange gazeux comprenant de 1 à 80 ppmv de NO, typiquement moins de 40 ppmv, et au moins 20 %vol. d'oxygène environ, de préférence au moins 21 %vol. d'oxygène environ, au moyen d'une installation de fourniture de gaz, telle celle décrite ci-avant selon l'invention, comprenant un appareil de délivrance de NO selon l'invention assurant une délivrance de NO, de manière à traiter (au moins partiellement) ladite hypertension pulmonaire et/ou ladite hypoxie, qui peut être causée par une (ou des) pathologie ou autres troubles pulmonaires typiquement de type PPHN (hypertension pulmonaire persistante du nouveau-né) ou SDRA (syndrome de détresse respiratoire aigüe), ou encore engendrée par une opération de chirurgie cardiaque avec mise du patient sous circulation sanguine extracorporelle (CEC).

Définitions

D'une façon générale, dans le cadre de l'invention :

- « ppmv » signifie partie par million en volume,
- « %vol. » » signifie pourcentage en volume.
- « NO » désigne le monoxyde d'azote.
- « NO$_2$ » désigne le dioxyde d'azote.
- « N$_2$ » désigne l'azote.
- « O$_2$ » désigne l'oxygène.

- les termes « concentration », « quantité », « proportion », « dose » et « teneur » sont considérés comme équivalents et substituables.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à vannes » peuvent être remplacés par « dispositif à vannes », les « moyens de mémorisation» peuvent être remplacés par « dispositif de mémorisation» ...
- par « mesure de débit », on entend une valeur de débit (e.g. une valeur numérique) ou un signal représentatif d'une telle valeur de débit reflétant ou correspondant à un débit gazeux mesuré par un capteur de débit, tel un capteur de débit massique.
- par « mesure de pression », on entend une valeur de pression (e.g. une valeur numérique) ou un signal représentatif d'une telle valeur de pression reflétant ou correspondant à la pression gazeuse mesurée par un capteur de pression.
- par « mesure de concentration », on entend une valeur de teneur en un composé gazeux donné, comme le NO, le $NO_2$ ou l'$O_2$, c'est-à-dire une valeur numérique ou un signal représentatif d'une telle valeur de teneur reflétant ou correspondant à la proportion (i.e. quantité) du composé gazeux considéré dans un mélange gazeux donné, mesurée par des moyens de mesure de concentration, telles des capteurs à cellules électrochimiques.

L'invention va maintenant être mieux comprise grâce à la description détaillée suivante, faite à titre illustratif mais non limitatif, en référence aux figures annexées parmi lesquelles :

Fig. 1 schématise un mode de réalisation d'une installation d'administration de gaz comprenant un appareil de délivrance de NO selon l'invention.
Fig. 2 schématise un mode de réalisation de l'architecture interne de l'appareil de délivrance de NO selon l'invention, tel celui de l'installation de Fig. 1.
Fig. 3 schématise un mode de réalisation de l'affichage de différentes informations sur l'afficheur d'un appareil de délivrance de NO selon l'invention, tel celui de Fig. 2, en particulier d'une première dose de NO ayant été réglée.
Fig. 4 schématise une modification de la dose de NO par l'utilisateur.
Fig. 5 est similaire à Fig. 3 mais schématise l'affichage obtenu après modification de la dose NO par l'utilisateur.

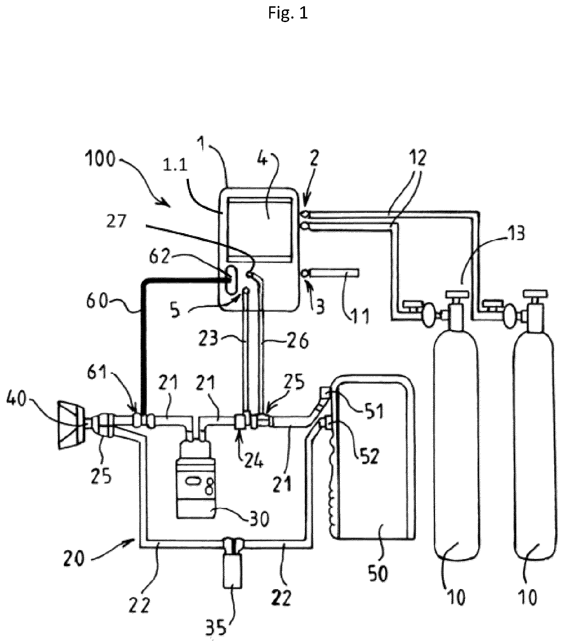

Fig. 1 schématise un mode de réalisation d'une installation d'administration de gaz 100 selon l'invention comprenant un appareil de délivrance de NO 1 selon l'invention permettant de fournir un mélange gazeux à base de monoxyde d'azote (NO), typiquement un mélange gazeux $NO/N_2$, et un ventilateur médical 50 fournissant un gaz contenant au moins 20%vol. d'oxygène, typiquement de l'air, un mélange gazeux $O_2/N_2$ ou autre.

L'installation 100 comprend ici deux bouteilles de gaz sous pression 10 contenant chacune un mélange gazeux à base de NO, à savoir un mélange gazeux $NO/N_2$ contenant ici entre 100 et 1000 ppmv de NO (reste $N_2$), par exemple 450 ou 800 ppm vol. de NO (reste $N_2$), ou toute autre concentration adéquate, qui alimentent en mélange $NO/N_2$, le dispositif ou appareil 1 de délivrance ou fourniture de NO permettant de suivre et contrôler la fourniture du mélange gazeux $NO/N_2$.

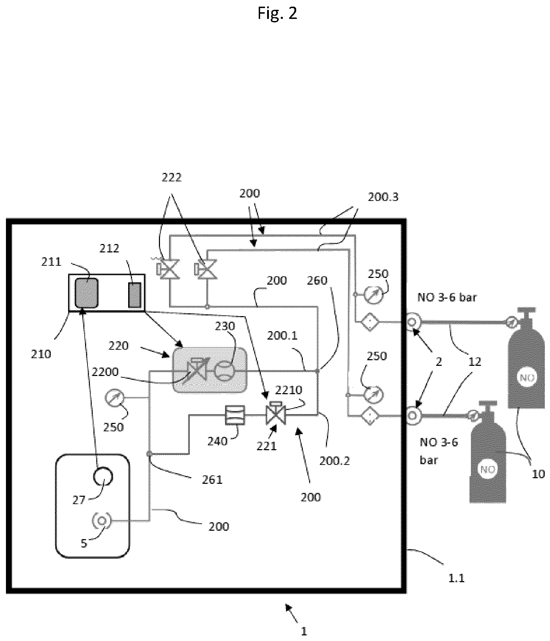

Les bouteilles de gaz 10 sont reliées fluidiquement à l'appareil 1 de fourniture de NO, via des lignes d'amenée de gaz 12, tel que des tuyaux ou conduits flexibles ou analogues, qui peuvent être équipées de dispositifs de régulation et/ou de suivi de la pression de gaz, tels que détendeur de gaz 13, manomètres... Les lignes d'amenée de gaz 12 sont reliées à une ou plusieurs entrées de gaz 2 de l'appareil de délivrance de NO 1 qui alimentent un circuit de gaz interne 200, comme schématisé en Fig. 2, servant à acheminer le gaz au sein de l'appareil de fourniture de NO 1, c'est-à-dire dans le boitier ou carcasse 1.1 externe de l'appareil 1 de délivrance de NO selon l'invention.

Dans le mode de réalisation de Fig. 2, le circuit de gaz interne 200 est relié à deux entrées de gaz 2 agencées en parallèle et alimentant chacune un tronçon d'entrée 200.3 dédié du circuit de gaz interne 200. Des vannes de contrôle 222 ou analogue contrôlent le passage du flux de $NO/N_2$ dans ces tronçon d'entrée 200.3.

L'appareil de délivrance de NO 1 comprend par ailleurs une entrée d'oxygène 3 reliée fluidiquement, via une ligne d'amenée d'oxygène 11, tel un tuyau flexible ou analogue, à une source d'oxygène (non montrée), par exemple une bouteille d'oxygène sous pression ou un réseau hospitalier, c'est-à-dire une canalisation d'alimentation en oxygène agencée dans un bâtiment hospitalier. Ceci permet d'alimenter le circuit de gaz interne 200 en oxygène quand cela est nécessaire.

Le ventilateur médical 50, c'est-à-dire un appareil d'assistance respiratoire, fournit un flux de gaz respiratoire à base d'oxygène, c'est-à-dire contenant au moins 20%vol. d'oxygène environ, préférentiellement au moins 21%vol. d'oxygène environ, tel de l'air ou un mélange oxygène/azote ($N_2/O_2$).

Le ventilateur médical 50 et l'appareil de fourniture de NO 1 de l'installation 100 sont en communication fluidique avec un circuit respiratoire 20, aussi appelé circuit patient, en particulier avec une ligne d'alimentation en gaz ou branche inspiratoire 21 du circuit respiratoire 20, qui sert à acheminer le flux gazeux vers l'interface respiratoire 40 fournissant le flux gazeux thérapeutique au patient, c'est-à-dire un mélange gazeux combiné, i.e. mélange final, contenant la posologie en NO souhaitée. Ce mélange gazeux combiné est obtenu par mélange du flux à base d'oxygène (e.g. air ou mélange

$O_2/N_2$) provenant du ventilateur médical 50 et du flux contenant le NO, i.e. le mélange gazeux $NO/N_2$, délivré par l'appareil de délivrance de NO 1.

Pour ce faire, l'appareil de délivrance de NO 1 fournit ou injecte le mélange $NO/N_2$ dans le circuit respiratoire 20 véhiculant le flux à base d'oxygène, via un conduit ou une ligne d'injection 23, reliant fluidiquement le circuit de gaz interne 200 de l'appareil de fourniture de NO 1 à un dispositif d'injection 24 agencé sur la ligne d'alimentation en gaz 21.

Le dispositif d'injection 24 est configuré pour opérer un mélange du gaz contenant du NO provenant de l'appareil de délivrance de NO 1 avec le flux de gaz respiratoire contenant de l'$O_2$ provenant du ventilateur 50 et acheminé par la branche inspiratoire 21 du circuit respiratoire 20, et obtenir un mélange gazeux combiné contenant du NO et de l'oxygène, c'est-à-dire le mélange gazeux final administré au patient.

Plus précisément, le dispositif d'injection 24 comprend une première entrée de gaz alimentée en flux de gaz respiratoire contenant de l'$O_2$ provenant du ventilateur médical 50, une seconde entrée de gaz alimentée en gaz contenant du NO, i.e. provenant de l'appareil de délivrance de NO 1, et une sortie de gaz fournissant le mélange gazeux combiné contenant du NO et de l'oxygène, obtenu par mélange, au sein du dispositif d'injection 24, du gaz contenant du NO avec le flux de gaz respiratoire contenant de l'$O_2$.

Autrement dit, le flux de $NO/N_2$ amené par la ligne d'injection 23 se mélange alors (grâce au dispositif d'injection 24) au flux de gaz à base d'oxygène (> 20% d'$O_2$), e.g. de l'air ou un mélange oxygène/azote, délivré par le ventilateur médical 50 et véhiculé par la branche inspiratoire 21 du circuit patient 20 de sorte d'obtenir un mélange final, i.e. le mélange combiné, à administrer au patient contenant essentiellement du NO à la posologie désirée, de l'azote ($N_2$) et de l'oxygène ($O_2$), et éventuellement des impuretés inévitables (e.g. argon, $CO_2$, $NO_2$, ....), c'est-à-dire un mélange gazeux final $NO/N_2/O_2$.

La branche inspiratoire 21 du circuit 20 comprend en outre un humidificateur de gaz 30 agencé en aval du dispositif d'injection 24. Il permet d'humidifier le flux de gaz final, e.g. le mélange gazeux combiné $NO/N_2/O_2$, avant qu'il ne soit administré par inhalation au patient à traiter, au moyen d'une interface respiratoire 40, telle une sonde d'intubation trachéale, un masque respiratoire ou analogue.

Une ligne de récupération des gaz expirés par le patient forme une branche expiratoire 22 du circuit patient 20. Elle est reliée fluidiquement à la branche inspiratoire 21 via une pièce de raccordement 25, telle une pièce en Y.

La branche inspiratoire 21 est, à son extrémité amont, raccordée fluidiquement à un port de sortie 51 du ventilateur médical 50, tel un connecteur, raccord ou analogue, de manière à récupérer et acheminer le gaz à base d'oxygène, typiquement de l'air ou mélange $N_2/O_2$ fourni par le ventilateur médical 50, alors que la branche expiratoire 22 véhiculant les gaz expirés est raccordée fluidiquement à un port d'entrée 52 du ventilateur médical 50, tel un connecteur, raccord ou analogue, de manière à retourner au ventilateur médical 50 tout ou partie du débit des gaz expirés par le patient. La branche expiratoire 22 peut comprendre un ou plusieurs composants optionnels, par exemple un dispositif d'élimination du $CO_2$ 35, i.e. un piège à $CO_2$, tel un bac à chaud ou autre, permettant d'éliminer le $CO_2$ présent dans les gaz expirés par le patient, un filtre ou autre.

Par ailleurs, un capteur de débit 25, par exemple du type à fil chaud ou à différentiel de pression, est agencé sur le circuit respiratoire 20, en particulier sur la branche inspiratoire 21, entre le ventilateur 50 et le dispositif d'injection 24. Le capteur de débit 25 est relié à un port de connexion au capteur 27, de l'appareil de délivrance de NO 1, via une (des) ligne de mesure de débit 26 venant se raccorder audit port de connexion au capteur 27. Il sert à mesurer le débit de gaz délivré par le ventilateur 50, tel de l'air ou $N_2/O_2$, circulant dans la branche inspiratoire 21, en amont du dispositif d'injection.

Ces mesures de débit opérées par le capteur de débit 25 permettent de contrôler ou réguler plus efficacement la délivrance du flux de NO (i.e. $N_2/O_2$) par l'appareil de délivrance de NO 1, en particulier le débit de NO, puisque les mesures de débit opérées par le capteur de débit 25 sont retournées, via la ligne de mesure de débit 26 (i.e. câbles électriques ou analogues) et le port de connexion au capteur 27, à des moyens de pilotage 210 à (micro)processeur de l'appareil de délivrance de NO 1, typiquement un contrôleur, qui traitent ces mesures de débit comme expliqué ci-après et illustré en Fig. 2. Le port de connexion au capteur 27 est relié électriquement aux moyens de pilotage 210 via une ou des liaisons électriques, par exemple des câbles électriques ou analogues.

L'appareil de fourniture de NO 1 comprend une carcasse rigide 1.1, par exemple en polymère, comprenant le circuit de gaz interne 200 sur Fig. 2, typiquement des lignes, passages ou conduits de gaz ou analogue, qui relie fluidiquement l'entrée (ou les entrées) de gaz 21 de l'appareil de fourniture de NO 1 à la ligne d'injection 23 de manière à convoyer le flux de gaz à base de NO entre eux.

Dans le mode de réalisation schématisé en Fig. 2, une portion du circuit de gaz interne 200 comprend deux tronçons de gaz agencés en parallèle, à savoir un tronçon principal 200.1 et un tronçon secondaire 200.2, dit tronçon de secours. Le tronçon principal 200.1 et le tronçon secondaire 200.2 se raccordent fluidiquement l'un à l'autre et au reste du circuit de gaz 200 en des sites de raccordement amont 260 et aval 261 situés, respectivement, en amont et en aval de moyens de contrôle de débit principaux et secondaires 220, 221.

En fonctionnement normal de l'appareil 1, le flux de $NO/N_2$ transite par le tronçon principal 200.1, alors qu'en cas de dysfonctionnement rendant les moyens de contrôle de débit principaux 220 non-opérationnels, le flux de $NO/N_2$ est dévié et transite alors par le tronçon de secours 200.2.

Bien entendu, selon un autre mode de réalisation (non montré), le circuit de gaz interne 200 pourrait être configuré différemment, par exemple comprendre une ligne de gaz unique en lieu et place des deux tronçons 200.1, 200.2. Toutefois, dans ce mode de réalisation, un dysfonctionnement des moyens de contrôle de débit principaux 220 ne pourrait pas être pris en compte et l'appareil 1 deviendrait alors non-fonctionnel.

D'une façon générale, les moyens de contrôle de débit principaux et secondaires 220, 221, tels des moyens à vanne principaux et secondaires 2200, 2210, schématisés en Fig. 2, i.e. un (ou des) dispositif à vanne(s), par exemple une (ou des) électrovanne proportionnelle pilotée par les moyens de pilotage 210, sont agencés sur le circuit de gaz interne 200, notamment sur les tronçons principal 200.1 et secondaire 200.2, et servent à contrôler ou ajuster le flux gazeux qui y circule en direction de la ligne d'injection 23, c'est-à-dire vers le dispositif d'injection 24 et ce, que ce soit en mode de fonctionnement normal ou en mode de secours.

De préférence, le tronçon principal 200.1 comprend électrovanne proportionnelle 220 et un capteur de débit additionnel 230, typiquement un contrôleur de débit massique ou MFC, alors que le tronçon secondaire 200.2 comprend une (ou des) électrovanne de type tout ou rien (TOR) 221, de préférence pilotée en mode pulsée.

Préférentiellement, les moyens de contrôle de débit principaux et secondaires 220, 221 de l'appareil de fourniture de NO 1 sont commandés, i.e. contrôlés, par les moyens de pilotage 210, c'est-à-dire un (ou des) dispositif de pilotage ou (micro)contrôleur, agencés dans le boitier 1.1 de l'appareil de fourniture de NO 1.

Typiquement, les moyens de pilotage 210 comprennent une (des) carte électronique comprenant un (ou plusieurs) microprocesseur(s) 211 mettant en œuvre un ou des algorithmes. Les moyens de pilotage 210 permettent notamment d'ajuster ou contrôler le débit de gaz à base de NO en pilotant tout ou partie des moyens à vanne 2200, 2210, typiquement d'ouvrir ou fermer une ou des (électro)vannes, pour obtenir un débit de gaz à base de NO, typiquement autoriser ou stopper le débit de gaz.

Bien entendu, les moyens de pilotage 210 permettent en outre d'opérer des calculs et/ou de contrôler ou commander tous les éléments électromécaniques de l'appareil 1, tels que les capteurs, les affichages ...

Comme expliqué ci-après, les moyens de pilotage 210 peuvent déterminer le débit de NO à fournir pour obtenir la teneur en NO souhaitée dans le mélange combiné, c'est-à-dire la posologie en NO désirée, à partir notamment d'une consigne de teneur en NO réglée et/ou fixée par l'utilisateur, c'est-à-dire d'une dose de NO ($D_{NO}$) réglée par l'utilisateur, à savoir une première dose de NO ($D_{NO1}$) ou ultérieurement une seconde dose de NO ($D_{NO2}$) comme expliqué ci-après, de la composition du mélange gazeux $NO/N_2$, en particulier de la teneur en NO dans ce mélange gazeux $NO/N_2$ qui peut être mémorisée par l'appareil 1, et d'une (ou des) mesure de débit opérée(s) par le capteur de débit 25 agencé sur la branche inspiratoire 21 et relié par une ligne de mesure de débit 26 au dispositif de fourniture de NO 1, en particulier aux moyens de pilotage 210, via le port de connexion au capteur 27.

Le circuit de gaz interne 200 de l'appareil de fourniture de NO 1 peut aussi comprendre d'autres éléments ou composants, en particulier un (ou des) capteur de pression 250, un (ou des) capteur de débit additionnel ou débitmètre et/ou des dispositifs à orifice calibré 240 ou autres. Ces autres éléments peuvent être agencés en amont et/ou en aval des moyens de contrôle de débit 220, 221, i.e. des moyens à vanne, par exemple on peut utiliser un capteur de débit additionnel pour déterminer le débit de gaz à base de NO circulant dans tout ou partie du circuit de gaz interne 200, notamment pour s'assurer qu'il est conforme au débit souhaité.

En Fig. 2, on voit que le tronçon principal 200.1 comprend un capteur de débit additionnel 230 agencé en amont des moyens de contrôle de débit 220, tels des moyens à vanne 2200, par exemple une (des) électrovanne, préférentiellement une électrovanne proportionnelle, contrôlant le passage de gaz dans le tronçon principal 200.1. Cet ensemble forme un contrôleur de débit massique (MFC).

Par ailleurs, le tronçon secondaire 200.2 comprend un dispositif à orifice calibré 240 agencé en aval de moyens de contrôle de débit secondaire 221, tels des moyens à vanne secondaire 2210, préférentiellement une (des) électrovanne, contrôlant le débit de passage de gaz dans le tronçon secondaire 200.2.

Avantageusement, l'électrovanne des moyens de contrôle de débit secondaire 221 est du type tout ou rien (TOR), c'est-à-dire pouvant adopter 2 positions « stables », à savoir une position ouverte laissant passer le flux gazeux et une position fermée empêchant toute circulation de flux gazeux.

Par ailleurs, le débitmètre ou capteur de débit additionnel 230 du MFC peut être du type à différentiel de pression, massique ou autre, et coopère avec les moyens de pilotage 210 pour leur fournir des mesures de débit du flux de $NO/N_2$.

Habituellement, l'appareil de fourniture de NO 1 comprend aussi une interface graphique utilisateur ou IGU comprenant un afficheur graphique 4, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections ou encore à entrer des informations, telles des valeurs désirées (e.g. débit, dosage de NO...), ou toute autre information ou donnée utile au personnel soignant. De préférence, l'affichage est en couleurs mais il peut se faire aussi en noir et blanc.

Toutefois, selon un autre mode de réalisation, l'appareil 1 peut aussi comprendre un ou des organes de sélection mécaniques, i.e. non-virtuels, comme un ou des boutons ou touches de sélection actionnable par pression digitale de l'utilisateur, un (ou des) bouton de sélection rotatif ou autre. Les sélections ou choix opérées via ce ou ces organes de sélection mécaniques peuvent s'afficher sur l'afficheur graphique 4 de l'IGU.

En particulier, les première dose de NO ($D_{NO1}$) et seconde dose de NO ($D_{NO2}$) peuvent être réglées ou sélectionnées via une ou des touches de sélection virtuelles et/ou des pavés ou fenêtres affichées par l'afficheur graphique 4, en particulier lorsqu'il est à dalle tactile.

L'alimentation électrique de l'appareil de fourniture de NO 1, en particulier des composants nécessitant du courant électrique pour fonctionner, tels les moyens de pilotage 210, l'afficheur graphique 4..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant. L'alimentation électrique du ventilateur médical 50 est assurée de façon analogue, notamment par une liaison au courant du secteur ou une batterie interne.

En outre, l'installation 100 comprend aussi une ligne de prélèvement de gaz 60 qui relie fluidiquement la branche inspiratoire 21 à l'appareil de fourniture de NO 1.

La ligne de prélèvement de gaz 60 permet d'assurer un monitorage, c'est-à-dire un suivi, de la composition du mélange gazeux combiné contenant le NO, l'$O_2$ etc... afin de s'assurer que les teneurs en NO, en $O_2$ et aussi en espèces toxiques $NO_2$ pouvant se former sont conformes, en particulier que la proportion de NO est bien comprise entre des seuils d'alarme NO haut et bas fixés, c'est-à-dire entre des teneurs de NO maximale et minimale acceptables. Dans le cas contraire, les moyens de pilotage de l'appareil 1 déclenchent une alarme sonore et/ou une alarme visuelle afin d'alerter le personnel soignant, en activant (i.e. pilotant/commandant) les moyens d'alarme.

La ligne de prélèvement de gaz 60, aussi appelée ligne de monitorage, vient se raccorder fluidiquement, en un site de raccordement 61 sur Fig. 1, à la ligne d'alimentation en gaz 21, entre l'humidificateur 30 et la pièce de jonction 25, i.e. pièce en Y, typiquement à proximité immédiate de la pièce de jonction 25, et par ailleurs à un port d'entrée 62 du dispositif de fourniture de NO 1, par exemple un port 62 porté par un connecteur, raccord ou analogue, permettant le raccordement de la ligne de prélèvement de gaz 60, tel un tuyau flexible ou analogue.

La ligne de prélèvement de gaz 60 permet de prélever des échantillons de gaz et de les convoyer jusqu'au dispositif de fourniture de NO 1 où ils sont analysés dans un analyseur de gaz interne (non montré), c'est-à-dire au sein d'une ligne de calibration comprenant au moins un capteur, notamment une ou des cellules électrochimiques, relié électriquement aux moyens de pilotage, afin de vérifier leur conformité.

Comme déjà dit, il convient de vérifier que la composition du gaz final, i.e. mélange gazeux combiné, est conforme à celle du mélange gazeux $NO/N_2/O_2$ souhaité devant être administré au patient, notamment pour s'assurer qu'il ne contient pas de quantité excessive d'espèces $NO_2$ toxiques, que sa teneur en oxygène n'est pas hypoxique, et que sa teneur en NO correspond à la posologie souhaitée (ni trop haute, ni trop basse), i.e. la dose de NO à administrer par inhalation qui est choisie par le personnel soignant, i.e. médecin ou analogue, à savoir la première dose de NO ($D_{NO1}$) ou la seconde dose de NO ($D_{NO2}$) en cas de modification de la première dose de NO ($D_{NO1}$).

Cette vérification de conformité se fait classiquement au moyen de moyens de mesure de concentration de NO, de $NO_2$ et d'$O_2$ dédiés, typiquement des capteurs de $NO_2$, de NO et d'$O_2$, par exemple des cellules électrochimiques ou analogues, qui doivent être calibrés périodiquement, par exemple toutes les semaines.

Les moyens de pilotage 210 de l'appareil 1 sont en outre configurés pour récupérer et traiter, i.e. analyser, les signaux provenant des différents moyens de mesure de concentration de NO, de $NO_2$ et d'$O_2$, tels des capteurs, de l'analyseur de gaz, lequel est agencé dans l'appareil 1, et d'agir en réponse à ces signaux, notamment pour opérer une calibration des capteurs, déclencher des alarmes sonore et/ou visuelle....

D'une façon générale, les moyens de pilotage 210 à (micro)processeur 211, tel un contrôleur, déterminent le débit de consigne de gaz contenant du NO à fournir au dispositif d'injection 24 et pilotent les moyens de contrôle de débit 220, 221, telles des électrovannes proportionnelles 220 et/ou TOR 221, pour fournir le gaz contenant du NO au débit de consigne. La détermination du débit de consigne de NO est réalisée à partir d'une mesure de débit de gaz respiratoire, i.e. valeur ou signal de débit, opérée et fournie par le capteur de débit 25 aux moyens de pilotage 210, d'une consigne de teneur en NO correspondant à la proportion finale de NO souhaitée dans le mélange gazeux combiné, typiquement fixée par l'utilisateur, i.e. personnel soignant, et de la proportion initiale de NO dans le gaz contenant du NO alimentant l'appareil de délivrance de NO 1, c'est-à-dire la quantité de NO présente dans le mélange $NO/N_2$ provenant des bouteilles de gaz 10, typiquement comprise entre 200 et 1000 ppmv, par exemple 450 ou 800 ppmv.

Préférentiellement, la teneur en NO dans le mélange gazeux $NO/N_2$ alimentant l'appareil 1 peuvent être mémorisées par les moyens de mémorisation 212 de l'appareil 1.

Par ailleurs, la teneur en NO dans le mélange gazeux $NO/N_2$ alimentant l'appareil 1 peut être ajustable ou non, c'est-à-dire mémorisée une fois pour toute ou réglable par l'utilisateur, au sein d'un menu dédié ou analogue de l'appareil 1, par exemple en utilisant, là encore, à des moyens de réglage, tels une ou des touches, boutons rotatifs, des curseurs ou autres.

La première dose de NO ($D_{NO1}$) désirée, qui est typiquement comprise entre 1 et 80 ppmv, c'est-à-dire la valeur de consigne de NO au départ d'un traitement, doit être entrée par l'utilisateur, par exemple via l'IHM, grâce à des moyens de réglage de dose ou analogue, tels une ou des touches, boutons rotatifs, des curseurs ou autres, de préférence via une ou des touches virtuelles s'affichant sur l'écran d'affichage 4.

Comme déjà expliqué, afin de pouvoir détecter et avertir l'utilisateur, c'est-à-dire le personnel soignant, en cas de concentration en NO excessive ou, à l'inverse, insuffisante dans le mélange gazeux combiné fourni au patient, il est indispensable de fixer les seuils d'alarme NO haut et bas, c'est-à-dire (au moins) un seuil haut et un seuil bas, correspondant aux teneurs de NO maximale et minimale acceptables compte tenu des fluctuations de concentration en NO pouvant exister du fait de variations possibles notamment du débit de gaz respiratoire à base d'oxygène provenant du ventilateur 50 médical.

Ainsi, juste avant le démarrage d'un traitement, par exemple lors de la mise en service de l'appareil 1, il convient de définir des premiers seuils d'alarme haut et bas correspondant à une première teneur maximale de NO ($T_{max1}$) et à une première teneur minimale de NO ($T_{min1}$), avec : $T_{max1} > D_{NO1} > T_{min1}$.

La fixation de ces seuils d'alarme NO haut et bas (i.e. $T_{max1}$, $T_{min1}$) se fait automatiquement, via les moyens de pilotage 210, dès réglage de la première dose de NO ($D_{NO1}$) désirée par l'utilisateur, i.e. un médecin par exemple, ce qui évite les erreurs de calcul et par ailleurs les erreurs liées à l'entrée de ces seuils d'alarme NO dans l'appareil de délivrance de NO. La sécurité des patients s'en trouve améliorée.

Plus précisément, pour ce faire, les moyens de pilotage 210 calculent ou déterminent automatiquement, à partir de la première dose de NO réglée ($D_{NO1}$) ayant été choisie par le médecin ou analogue, les premiers seuils d'alarme haut et bas correspondant à une première teneur maximale de NO ($T_{max1}$) et à une première teneur minimale de NO ($T_{min1}$), avec : $T_{max1} > D_{NO1} > T_{min1}$.

Une fois que la première dose de NO ($D_{NO1}$) désirée, i.e. posologie souhaitée, typiquement comprise entre 1 et 80 ppmv, de préférence inférieure ou égale à 60 ppmv, plus préférentiellement inférieure ou égale à 40 ppmv, par exemple de l'ordre de 20 ppmv, a été réglée par l'utilisateur en utilisant les moyens de réglage de dose de NO, telles une ou des touches virtuelles affichées par l'afficheur graphique 4, la valeur choisie correspondant à la première dose de NO ($D_{NO1}$) réglée, est utilisée par les moyens de pilotage 210 pour calculer ou déterminer automatiquement des premiers seuils d'alarme NO haut et bas comme suit :

- le premier seuil d'alarme NO haut correspond à une première teneur maximale de NO ($T_{max1}$), telle que : $1,1 . D_{NO1} \leq T_{max1} \leq 1,2.D_{NO1}$, par exemple : $T_{max1} = 1,2.D_{NO1}$, et
- le premier seuil d'alarme NO bas correspond à une première teneur minimale de NO ($T_{min1}$), telle que : $0,7 . D_{NO1} \leq T_{min1} \leq 0,80 . D_{NO1} \leq T_{min1}$, par exemple :

$$T_{min1} = 0,8.D_{NO1}.$$

Autrement dit, avant ou lors du démarrage d'un traitement, les moyens de pilotage 210 de l'appareil 1 déterminent automatiquement les premières valeurs des seuils d'alarme NO haut et bas ($T_{max1}$, $T_{min1}$) en ajoutant ou, à l'inverse, retranchant de 10% à 20% (i.e. une tolérance), de préférence 20%, à la valeur de première dose de NO ($D_{NO1}$) réglée, en fonction de la valeur de tolérance (%) que l'on peut accepter, c'est-à-dire des fluctuations de teneurs en NO dans le mélange gazeux combiné qui peuvent être tolérées dans le cadre du traitement du patient.

La valeur de tolérance (%) peut être non-modifiable, par exemple fixée en usine et mémorisée dans l'appareil, ou, selon un autre mode de réalisation, être modifiable par l'utilisateur, par exemple au sein d'un menu de configuration de l'appareil 1. Le pourcentage (%) de tolérance peut dépendre de différents facteurs, par exemple pour d'un protocole de traitement particulier. Avantageusement, la tolérance est fixée à 20%.

La valeur de tolérance (%) et/ou les formules de calcul incluant cette valeur de tolérance (%) sont mémorisées au sein de l'appareil 1, par exemple au sein des moyens de mémorisation 212. Par exemple une tolérance de 20% qui est appliquée au calcul de tous les seuils d'alarme de NO.

**[0089]** Une fois déterminées, la première teneur maximale de NO ($T_{max1}$) correspondant au premier seuil d'alarme NO haut et la première teneur minimale de NO ($T_{min1}$) correspondant au premier seuil d'alarme NO bas sont préférentiellement mémorisées, i.e. enregistrées, par les moyens de mémorisation 212.

Par exemple, pour une première dose de NO ($D_{NO1}$) réglée à 20 ppmv par le médecin ou analogue, l'appareil de délivrance 1 de l'invention, typiquement ses moyens de pilotage 210, calcule automatiquement des premiers seuils d'alarme NO haut et bas compris entre 14 et 18 ppmv pour la première teneur minimale de NO ($T_{min1}$) et entre 22 et 26 ppmv pour la première teneur maximale de NO ($T_{max1}$).

Si le pourcentage (%) de tolérance a été fixé à 20% du fait de réglages opérés par exemple en usine, les formules de calcul ci-dessus se résument alors à :

- $T_{max1} = 1,20.D_{NO1}$, pour le premier seuil d'alarme NO haut, soit 20% de plus que la première dose de NO ($D_{NO1}$) réglée, et

- $T_{min1}$ = 0,80. $D_{NO1}$ pour le premier seuil d'alarme NO bas, soit 20% de moins que la première dose de NO ($D_{NO1}$) réglée.

Dès lors, pour la première dose de NO ($D_{NO1}$) réglée à 20 ppmv, l'appareil 1 peut alors calculer automatiquement des premiers seuils d'alarme NO haut et bas égaux à 16 ppmv pour la première teneur minimale de NO ($T_{min1}$) et à 24 ppmv pour la première teneur maximale de NO ($T_{max1}$), c'est-à-dire des premiers seuils haut et bas égaux à 20 ppmv +/- 20% (i.e. +/- 4 ppmv).

Dans tous les cas, en particulier pour les doses de NO ($D_{NO}$) réglées qui sont inférieures à 10 ppmv, les moyens de pilotage 210 sont configurés pour calculer et/ou fixer des premières teneurs maximale et minimale de NO ($T_{max1}$, $T_{min1}$) s'écartant préférentiellement d'au moins 2 ppmv de la première dose de NO ($D_{NO1}$) réglée, c'est-à-dire que : $T_{max1}$ - $D_{NO1}$ ≥ 2 ppmv et $D_{NO1}$ - $T_{min1}$ ≥ 2 ppmv.

Par exemple, pour une première dose de NO ($D_{NO}$) réglée de 5 ppmv et une tolérance de 20%, les moyens de pilotage 210 sont configurés pour calculer et/ou fixer des premières teneurs maximale et minimale de NO ($T_{max1}$, $T_{min1}$) de 3 ppmv pour la première teneur minimale de NO ($T_{min1}$) et de 7 ppmv pour la première teneur maximale de NO ($T_{max1}$), c'est-à-dire de +/- 2 ppmv par rapport à la dose de NO ($D_{NO}$) de 5 ppmv, alors qu'en théorie, elles devraient être de 4 ppmv et 6 ppmv, respectivement, c'est-à-dire de +/- 20% par rapport à la dose de NO ($D_{NO}$) de 5 ppmv.

En effet, un tel écart d'au moins 2 ppmv de la dose de NO ($D_{NO}$) réglée permet d'éviter des déclenchements d'alarme trop fréquents tout en garantissant une bonne sécurité pour le patient.

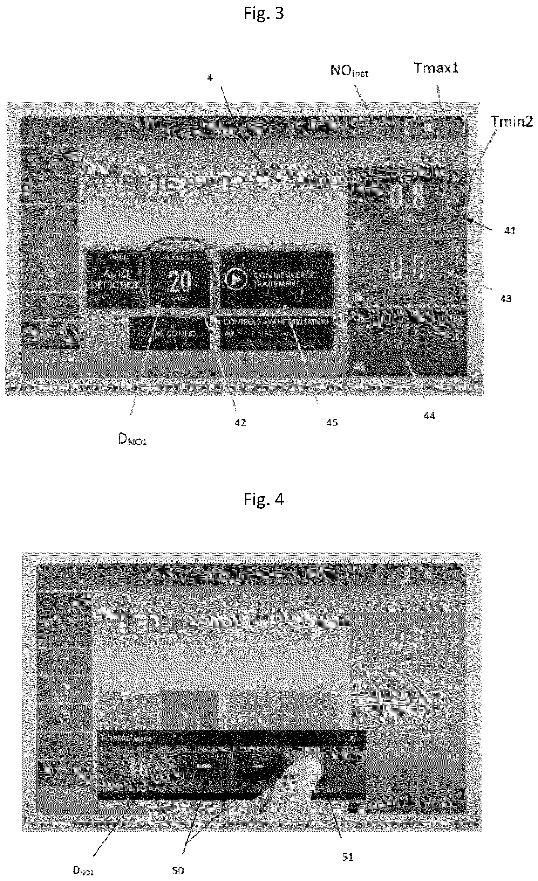

Une fois les premiers seuils d'alarme NO haut et bas déterminés, ils sont préférentiellement affichés par l'afficheur graphique 4, comme illustré en Fig. 3, qui schématise un affichage dans une première fenêtre 41 dédiée, de la première teneur maximale de NO ($T_{max1}$) correspondant au premier seuil d'alarme NO haut, à savoir ici 24 ppmv, et de la première teneur minimale de NO ($T_{min1}$) correspondant au premier seuil d'alarme NO bas, à savoir ici 16 ppmv.

On voit qu'est affichée aussi dans la première fenêtre 41, la valeur instantanée de teneur en NO ($NO_{inst}$), à savoir ici 0,8 ppmv, qui est mesurée par les moyens de mesure de concentration de NO, tel un capteur de NO. Dans ce cas, le traitement n'ayant pas encore commencé (information affichée : ATTENTE - Patient Non-Traité), la valeur de NO dans le flux envoyé au patient est quasi-nulle (0,8 ppmv) car le flux de gaz respiratoire provenant du ventilateur médical 50 n'a pas encore été mélangée avec le flux de mélange NO/$N_2$ provenant de l'appareil 1. Dès lors, les moyens de mesure de concentration de NO n'en déterminent qu'en quantité négligeable dans le flux d'air, c'est-à-dire sous forme de traces ou d'impuretés inévitables.

Fig. 3 illustre donc l'afficheur 4 après fixation de la première dose de NO ($D_{NO}$) par l'utilisateur et calcul des premiers seuils d'alarme ($T_{max1}$, $T_{min1}$) mais juste avant le début du traitement du patient, qui peut alors débuter après appui par l'utilisateur sur la touche virtuelle de démarrage 45 s'affichant sur l'afficheur graphique 4. D'ailleurs, on voit aussi que la première dose de NO ($D_{NO1}$) réglée, c'est-à-dire la posologie, est aussi affichée au sein d'une seconde fenêtre 42, à savoir ici 20 ppmv.

Les moyens de mesure de concentration de NO fournissent les mesures de NO (i.e. valeur ou signal) aux moyens de pilotage 210 qui les traitent pour déclencher éventuellement des alarmes et/ou contrôlent leur affichage sur l'afficheur 4 et ce, afin d'assurer un monitorage efficace du fonctionnement de l'appareil 1 et/ou de l'installation 100.

De plus, l'afficheur 4 affiche aussi les concentrations en $NO_2$ et $O_2$ mesurées par les moyens de mesure de concentration de $NO_2$ et d'$O_2$, typiquement des capteurs de $NO_2$ et d'$O_2$, par exemple des cellules électrochimiques ou analogues, et traitées par les moyens de pilotage 210, lesquels contrôlent également leur affichage sur l'afficheur 4, à savoir ici dans des troisième et quatrième fenêtres 43, 44.

On voit que la teneur en $NO_2$ mesurée et affichée dans la troisième fenêtre 43 est égale ici à 0 ppmv, ce qui est normal puisque le traitement n'ayant pas encore commencé, donc aucune oxydation des molécules de NO pour former les espèces toxiques $NO_2$ n'a pu avoir eu lieu.

Par contre, on constate que la fourniture du flux de gaz respiratoire contenant de l'oxygène, tel de l'air ou un mélange gazeux formé de 21% $O_2$ et reste $N_2$ par exemple, provenant du ventilateur 50 a débutée puisque les moyens de mesure de concentration d'$O_2$ ont détecté une teneur en oxygène de 21%vol. environ dans le flux gazeux présent dans le circuit respiratoire 20. Cette teneur en oxygène est affichée dans la quatrième fenêtre 44.

Une fois la première dose de NO réglée ($D_{NO1}$) par l'utilisateur et les premiers seuils d'alarme haut et bas correspondant aux premières teneurs maximale de NO ($T_{max1}$) et minimale de NO ($T_{min1}$) calculés par l'appareil 1, par exemple 16 et 24 ppmv sur Fig. 3 pour une dose de 20 ppmv initialement fixée ou sélectionnée par l'utilisateur, le traitement d'un patient peuvent être lancé par l'utilisateur, i.e. le personnel soignant.

Le NO est dès lors distribué par l'appareil 1 à un débit donné permettant d'obtenir la posologie de NO désirée dans le mélange gazeux combiné, à savoir une posologie correspondant à la première dose de NO réglée ($D_{NO1}$) par l'utilisateur. Ceci se fait par pilotage de tout ou partie des moyens de contrôle de débit 220, 221, comme déjà expliqué.

Toutefois, si le médecin ou analogue se rend compte que la dose de NO fournie ne convient pas au patient, c'est-à-dire qu'elle est trop forte ou trop faible, alors il peut modifier cette première dose de NO ($D_{NO1}$), via des moyens de modification de dose de NO, à savoir comme précédemment une ou des touches virtuelles par exemple, pour adopter une seconde dose de NO ($D_{NO2}$) différente de la première dose de NO ($D_{NO1}$).

Par exemple, si la première dose de NO ($D_{NO1}$) ayant été réglée, c'est-à-dire la posologie de départ, était de 20 ppmv, comme illustré sur Fig. 3, et que le médecin pense qu'elle est trop forte pour le patient considéré, il peut décider de la diminuer par exemple à 16 ppmv (ou à l'inverse, de l'augmenter si trop faible).

Dans ce cas, comme schématisé en Fig. 4, il actionne des moyens de modification de dose de NO, telle une ou des touches virtuelles affichées par l'afficheur graphique 4 à dalle tactile, par exemple des touches « + » ou « - », servant à incrémenter ou décrémenter la valeur affichée par l'afficheur graphique 4 de manière à modifier la première dose de NO ($D_{NO1}$), à savoir ici 20 ppmv par exemple, pour obtenir ou fixer la seconde dose de NO ($D_{NO2}$) désirée, à savoir ici 16 ppmv par exemple.

Une fois la seconde dose de NO ($D_{NO2}$) fixée, il peut la valider via une touche de validation 51 par exemple, qui est activée par appui digital de l'utilisateur.

Selon un autre mode de réalisation (non montré), la (ou les) dose de NO pourrait aussi être directement entrée via un pavé numérique virtuel ou réel de l'interface graphique utilisateur (IGU) de l'appareil ou encore sélectionnée au sein d'une liste de valeurs possibles et sélectionnables.

Dès lors, selon l'invention, les moyens de pilotage 210 sont configurés pour calculer automatiquement, à partir de ladite seconde dose de NO ($D_{NO2}$), i.e. ici 16 ppmv, des seconds seuils d'alarme NO haut et bas ($T_{max2}$, $T_{min2}$) différents des premiers seuils d'alarme NO haut et bas ($T_{max1}$, $T_{min1}$), correspondant à des secondes teneurs maximale de NO ($T_{max2}$) et minimale de NO ($T_{min2}$), avec :

$$T_{max2} > D_{NO2} > T_{min2}.$$

Le calcul de ces seconds seuils d'alarme NO haut et bas ($T_{max2}$, $T_{min2}$) se fait comme celui des premiers seuils d'alarme NO haut et bas ($T_{max1}$, $T_{min1}$), comme expliqué ci-avant, et ce, avec la même valeur de tolérance, à savoir de 10 à 20%, de préférence de 20%.

Ainsi, si pour une seconde dose de NO ($D_{NO2}$) réglée à 16 ppmv (pour une première dose de NO ($D_{NO1}$) précédemment réglée à 20 ppmv) et une tolérance fixée à 20%, l'appareil 1 peut calculer automatiquement des secondes seuils d'alarme NO haut et bas égaux à 13 ppmv pour la seconde teneur minimale de NO ($T_{min2}$) et à 19 ppmv pour la seconde teneur maximale de NO ($T_{max2}$), c'est-à-dire des seconds seuils haut et bas égaux à 16 ppmv +/- 20% (i.e. +/- 3 ppmv).

Toutefois, comme précédemment, pour les doses de NO inférieures à 10 ppmv, les moyens de pilotage 210 sont configurés pour calculer et/ou fixer des secondes teneurs maximale et minimale de NO ($T_{max2}$, $T_{min2}$) s'écartant préférentiellement d'au moins 2 ppmv de la seconde dose de NO ($D_{NO2}$) réglée, de préférence de +/- 2 ppmv.

Comme déjà expliqué, observer un tel écart minimum d'au moins 2 ppmv par rapport à la dose de NO ($D_{NO}$) réglée, de préférence un écart égale à +/- 2 ppmv, pour une dose de moins de 10 ppmv permet d'éviter des déclenchements d'alarme intempestifs car trop fréquents, tout en garantissant une bonne sécurité pour le patient, en particulier pour les patients pédiatriques, typiquement les nouveau-nés, les bébés, les petits enfants ou analogue, qui doivent recevoir des doses de NO plus faibles que les patients adultes.

On comprend dès lors que, dans ce cas, un contrôle strict des seuils d'alarme en cas de modification de la dose dispensée est indispensable pour éviter des problèmes de dosage tout en garantissant quel le traitement par NO qui est administré au patient se fait de manière efficace.

Comme dans le cadre de l'invention, le re-calcul des seuils d'alarme en cas de modification de la dose de NO à administrer, se fait de manière automatique et instantanée, c'est-à-dire en temps réel, dès validation de la nouvelle dose de NO à administrer, à savoir de la seconde dose de NO ($D_{NO2}$), il est indispensable que les moyens de pilotage puissent déterminer si la nouvelle dose est inférieure à 10 ppmv et, si tel est le cas, agir en réponse en calculant les nouveaux seuils d'alarme en respectant la condition susmentionnée, à savoir un écart de +/- 2 ppmv de la nouvelle dose choisie par l'utilisateur, i.e. médecin ou analogue.

Le fait que ce re-calcul soit fait par l'appareil lui-même permet d'éviter des erreurs d'entrée ou analogue, ce qui améliore encore plus la sécurité du patient.

Observer cet écart d'au moins 2 ppmv pour les doses de NO de moins de 10 ppmv, de préférence de +/-2 ppmv, est particulièrement important lors des recalculs de dose car c'est à ce moment que la nouvelle dose de NO choisie est susceptible de passer sous 10 ppmv, typiquement lorsque le personnel soignant s'aperçoit que la dose initiale est trop forte. En effet, la dose de départ choisie dans nombre de services de soins hospitaliers est généralement d'au moins 10 ppmv, par exemple de 10, 15 ou 20 ppmv, et le calcul des seuils haut et bas se fait alors à +/- 10% à 20% comme expliqué ci-avant.

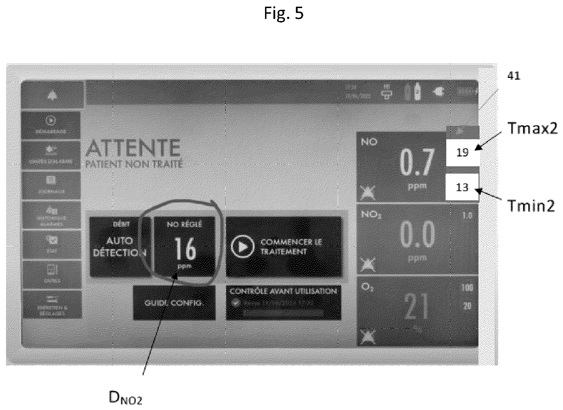

Ensuite, comme illustré en Fig. 5, les affichages opérés sur l'afficheur graphique 4 changent, c'est-à-dire se réactualisent, pour afficher non seulement la « nouvelle » ou seconde dose de NO ($D_{NO2}$) mais aussi les « nouvelles » ou secondes teneurs maximale et minimale de NO ($T_{min2}$, $T_{max2}$) correspondant aux seconds seuils d'alarme NO haut et bas ayant été déterminés automatiquement à partir de la seconde dose de NO ($D_{NO2}$) choisie par l'utilisateur.

Bien entendu, si l'utilisateur venant à encore changer la posologie de NO, c'est-à-dire d'appliquer une troisième dose de NO (voire d'autres doses de NO, par exemple une quatrième, une cinquième...), alors l'appareil 1 opérerait de la même manière pour calculer de « nouvelles » ou troisièmes (ou plus) teneurs maximale et minimale de NO ($T_{min3}$, $T_{max3}$).

Dans tous les cas, le fait de programmer l'appareil 1 pour qu'il réactualise automatiquement les niveaux des alarmes en cas de changement de dose de NO, c'est-à-dire de posologie, est avantageux car il évite le déclenchement d'alarmes intempestives qui pourraient se produire en cas d'oubli par l'utilisateur d'opérer une telle adaptation des niveaux d'alarme ou dans le cas, où il se tromperait en calculant les niveaux des nouvelles alarmes et entrerait alors des valeurs de seuils erronées.

Ensuite, pendant le fonctionnement de l'appareil 1, lorsque les moyens de pilotage 210 détectent une concentration de NO dans le mélange combiné $NO/O_2/N_2$ supérieure à la première teneur maximale de NO ($T_{max1}$) ou, en cas de changement de teneur en NO, à une seconde teneur maximale de NO ($T_{max2}$), c'est-à-dire excédant le seuil haut ($T_{max1}$, $T_{max2}$), ou, à l'inverse, inférieure à la première teneur minimale de NO ($T_{min1}$), ou, en cas de changement de teneur en NO, à une seconde teneur minimale de NO ($T_{min2}$), c'est-à-dire excédant le seuil bas ($T_{min1}$, $T_{min2}$), ils agissent sur des moyens d'alarme de l'appareil 1 pour déclencher une alarme, qui peut être sonore, par exemple un son provenant d'un buzzer ou analogue, et/ou visuelle, par exemple un message d'alerte s'affichant sur l'afficheur graphique 4, afin d'avertir le personnel soignant que la teneur en NO du mélange gazeux combiné est trop élevée ou, à l'inverse, trop faible.

Bien entendu, d'autres alarmes peuvent être déclenchées par les moyens de pilotage 210 en cas de détection d'une teneur en $NO_2$ trop élevée, par exemple excédant 1 à 3 ppmv environ ou d'une teneur en oxygène trop faible, par exemple inférieure à 19 à 20 %vol environ. Ces alarmes peuvent être fixées en usine et/ou optionnellement, être modifiables par l'utilisateur.

Une installation d'administration de gaz 100 incluant un appareil de délivrance de NO 1 selon l'invention comprenant des moyens de modification automatique des seuils d'alarmes, lors des changements de dose, peut être utilisée pour administrer par inhalation du monoxyde d'azote (NO), i.e. le mélange final obtenu $NO/O_2/N_2$, aux personnes, i.e. patients, souffrant d'hypertension artérielle pulmonaire aiguë, notamment pour opérer une dilatation de leurs vaisseaux pulmonaires et une augmentation de leur oxygénation en améliorant les échanges gazeux pulmonaires, en particulier pour traiter l'Hypertension Artérielle Pulmonaire du Nouveau-né ou PPHN, le Syndrome de Détresse Respiratoire Aigüe ou SDRA observé principalement chez l'adulte, ou les hypertensions pulmonaires (HP) en chirurgie cardiaque chez l'adulte ou l'enfant.

## Revendications

**1.** Appareil de délivrance (1) d'un gaz contenant du NO comprenant :

- des moyens de réglage de dose de NO (50, 51) configurés pour permettre à un utilisateur de régler une première dose de NO ($D_{NO1}$) comprise entre 1 et 80 ppmv, et
- des moyens de pilotage (210) à microprocesseur (211) configurés pour déterminer à partir de ladite première dose de NO réglée ($D_{NO1}$), des premiers seuils d'alarme haut et bas correspondant à une première teneur maximale de NO ($T_{max1}$) et à une première teneur minimale de NO ($T_{min1}$), avec :

$$T_{max1} > D_{NO1} > T_{min1},$$

et
- des moyens de modification de dose de NO (50, 51) configurées pour permettre à un utilisateur de modifier ou ajuster la première dose de NO ($D_{NO1}$) pour obtenir ou fixer une seconde dose de NO ($D_{NO2}$) différente de la première dose de NO ($D_{NO1}$), et

**caractérisé en ce que** les moyens de pilotage (210) sont configurés pour déterminer automatiquement à partir de ladite seconde dose de NO ($D_{NO2}$), des seconds seuils d'alarme NO haut et bas différents desdits premiers seuils d'alarme NO haut et bas, correspondant à une seconde teneur maximale de NO ($T_{max2}$) et à une seconde teneur minimale de NO ($T_{min2}$), avec :

$$T_{max2} > D_{NO2} > T_{min2},$$

où :

$$T_{max2} \leq 1{,}20 \cdot D_{NO2} \quad \text{et} \quad 0{,}80 \cdot D_{NO2} \leq T_{min2}$$

et, lorsque la seconde dose de NO ($D_{NO2}$) est inférieure à 10 ppmv, avec : $T_{max2}$ - $D_{NO2} \geq 2$ ppmv et $D_{NO2}$ - $T_{min2} \geq 2$ ppmv.

2. Appareil selon la revendication 1, **caractérisé en ce que** les moyens de pilotage (210) sont en outre configurés pour calculer et/ou fixer des premières teneurs maximale et minimale de NO ($T_{max1}$, $T_{min1}$) telles que :

$$T_{max1} \leq 1,20 \cdot D_{NO1} \text{ et } 0,80 \cdot D_{NO1} \leq T_{min1},$$

et avec : $T_{max1}$ - $D_{NO1} \geq 2$ ppmv et $D_{NO1}$ - $T_{min1} \geq 2$ ppmv, lorsque la dose de NO ($D_{NO}$) réglée est inférieure à 10 ppmv.

3. Appareil selon l'une des revendications précédentes, **caractérisé en ce qu'**il comprend en outre un afficheur graphique (4) configuré pour afficher au moins :

- la première dose de NO réglée ($D_{NO1}$) et les premières teneurs maximale et minimale de NO ($T_{min1}$, $T_{max1}$) correspondant aux premiers seuils d'alarme haut et bas, ou
- la seconde dose de NO ($D_{NO2}$) et les secondes teneurs maximale et minimale de NO ($T_{min2}$, $T_{max2}$) correspondant aux seconds seuils d'alarme NO haut et bas ayant été déterminés.

4. Appareil selon l'une des revendications précédentes, **caractérisé en ce que** les moyens de pilotage (210) sont configurés pour déterminer les secondes teneurs minimale et maximale de NO ($T_{min2}$, $T_{max2}$) telles que :

- lorsque : $D_{NO2} < D_{NO1}$ alors : $T_{max2} < T_{max1}$ et $T_{min2} < T_{min1}$, ou
- lorsque : $D_{NO2} > D_{NO1}$ alors : $T_{max2} > T_{max1}$ et $T_{min2} > T_{min1}$.

5. Appareil selon la revendication 1, **caractérisé en ce qu'**il comprend en outre un circuit de gaz interne (200) pour acheminer un flux de gaz contenant du NO, comprenant des moyens de contrôle de débit (220, 221) configurés pour contrôler le flux de gaz contenant du NO au sein dudit circuit de gaz (200).

6. Appareil selon la revendication 5, **caractérisé en ce que** les moyens de pilotage (210) à microprocesseur (211) sont configurés pour piloter au moins les moyens de contrôle de débit (220, 221) pour contrôler la fourniture de gaz contenant du NO en fonction d'au moins ladite première dose de NO réglée ($D_{NO1}$) ou de la seconde dose de NO ($D_{NO2}$) réglée.

7. Appareil selon la revendication 5, **caractérisé en ce que** :

- le circuit de gaz interne (200) de l'appareil (1) véhiculant le gaz contenant le NO est raccordé fluidiquement à un circuit respiratoire (20 ; 21) véhiculant un gaz respiratoire contenant de l'oxygène pour y injecter le gaz contenant le NO et obtenir un mélange gazeux combiné contenant du NO et de l'oxygène,
- et les moyens de pilotage (210) sont configurés pour déclencher une alarme en activant des moyens d'alarme, lorsque la teneur en NO dans le mélange gazeux combiné est supérieure ou égale à la première ou, selon le cas, à la seconde teneur maximale de NO ($T_{max1}$, $T_{max2}$) ou inférieure ou égale à la première ou, selon le cas, à la seconde teneur minimale de NO ($T_{min1}$, $T_{min2}$).

8. Appareil selon les revendications 3 et 7, **caractérisé en ce que** des moyens de mesure de concentration de NO sont agencés de manière à déterminer la teneur en NO dans le mélange gazeux combiné au sein du circuit de gaz interne (200) et les moyens de pilotage (210) sont configurés pour commander un affichage (41) sur l'afficheur graphique (4) de la teneur en NO ($NO_{inst}$) dans le mélange gazeux combiné ayant été déterminée par des moyens de mesure de concentration de NO.

9. Appareil selon les revendications 1 et 3, **caractérisé en ce que** les moyens de réglage de dose de NO ou de modification de dose de NO (50, 51) comprennent une ou plusieurs touches virtuelles affichées sur l'afficheur graphique (4).

10. Appareil selon la revendication 1, **caractérisé en ce que**, lorsque la seconde dose de NO ($D_{NO2}$) est inférieure à 10 ppmv, les moyens de pilotage (210) sont configurés pour déterminer automatiquement les seconds seuils d'alarme NO haut et bas correspondant aux secondes teneurs maximale et minimale de NO ($T_{max2}$, $T_{min2}$), tels que : $T_{max2} = D_{NO2} + 2$ ppmv et $T_{min2} = D_{NO2} - 2$ ppm.

**11.** Appareil selon la revendication 1, **caractérisé en ce que** la seonde dose de NO ($D_{NO2}$) comprise entre 1 et 80 ppmv.

**12.** Installation de fourniture d'un gaz contenant du NO (100) comprenant l'appareil de délivrance de NO (1) selon l'une des revendications précédentes alimenté en un mélange gazeux NO/$N_2$ par une source de mélange NO/$N_2$, et un ventilateur médical (50) configuré pour fournir un flux de gaz respiratoire contenant de l'$O_2$.

**13.** Installation selon la revendication 12, **caractérisée en ce que** le ventilateur médical (50) est configuré pour fournir un flux de gaz respiratoire contenant au moins 20%vol. environ d'$O_2$, typiquement un mélange gazeux NO/$N_2$ ou de l'air.

**14.** Installation selon la revendication 12, **caractérisée en ce que** l'appareil de délivrance de NO (1) et le ventilateur médical (50) sont raccordés fluidiquement au circuit respiratoire (20 ; 21) pour lui fournir des flux gazeux.

**15.** Installation selon la revendication 12, **caractérisée en ce qu'**un capteur de débit (25) est agencé dans le circuit respiratoire (20) entre le ventilateur médical (50) et un dispositif d'injection (24).

Fig. 1

Fig. 2

Fig. 3

Fig. 4

22

Fig. 5

Europäisches Patentamt
European Patent Office
Office européen des brevets

# RAPPORT DE RECHERCHE EUROPEENNE

Numéro de la demande

EP 25 16 4536

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | US 2022/106189 A1 (MILLER J W RANDOLPH [US]) 7 avril 2022 (2022-04-07) * le document en entier, et en particulier le paragraphe [0168] et les figures 1 à 5; voir également les paragraphes [0104]-[0105], [0108], [0116]-[0120], [0139]-[0142], [0161] et [0163] * ----- | 1-15 | INV. A61M16/00 A61M16/10 ADD. A61M16/12 A61M16/20 |
| A,D | US 11 833 309 B2 (THIRD POLE INC [US]) 5 décembre 2023 (2023-12-05) * le document en entier, et en particulier la colonne 49 aux lignes 27 à 32, la colonne 56 aux lignes 21 à 60, et les figures 51A à 51D * ----- | 1-15 | |
| A,D | US 2013/118486 A1 (SCHNITMAN ROBERT [US] ET AL) 16 mai 2013 (2013-05-16) * le document en entier, et en particulier le paragraphe [0044] et les figures 2 et 5 * ----- | 1-15 | |
| A,D | US 2013/192595 A1 (TOLMIE CRAIG R [US] ET AL) 1 août 2013 (2013-08-01) * le document en entier, et en particulier les paragraphes [0017]-[0019] et [0049]-[0051], les revendications 13 à 16, et les figures 2 et 5 * ----- | 1-15 | DOMAINES TECHNIQUES RECHERCHES (IPC) A61M |

Le présent rapport a été établi pour toutes les revendications

| Lieu de la recherche | Date d'achèvement de la recherche | Examinateur |
|---|---|---|
| Munich | 25 avril 2025 | Azaïzia, Mourad |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 25 16 4536

La présente annexe indique les membres de la famille de brevets relatifs aux documents  brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-04-2025

| Document brevet cité au rapport de recherche | Date de publication | Membre(s) de la famille de brevet(s) | Date de publication |
|---|---|---|---|
| US 2022106189 A1 | 07-04-2022 | AUCUN | |
| US 11833309 B2 | 05-12-2023 | US 2022339391 A1 | 27-10-2022 |
| | | US 2023263986 A1 | 24-08-2023 |
| | | US 2024325672 A1 | 03-10-2024 |
| US 2013118486 A1 | 16-05-2013 | AU 2012335937 A1 | 19-06-2014 |
| | | CA 2854776 A1 | 16-05-2013 |
| | | EP 2776106 A1 | 17-09-2014 |
| | | EP 4154930 A1 | 29-03-2023 |
| | | HK 1198520 A1 | 15-05-2015 |
| | | JP 6905447 B2 | 21-07-2021 |
| | | JP 7100009 B2 | 12-07-2022 |
| | | JP 2014532523 A | 08-12-2014 |
| | | JP 2018038836 A | 15-03-2018 |
| | | JP 2020044373 A | 26-03-2020 |
| | | MX 362813 B | 08-02-2019 |
| | | US 2013118486 A1 | 16-05-2013 |
| | | US 2017348502 A1 | 07-12-2017 |
| | | WO 2013070712 A1 | 16-05-2013 |
| US 2013192595 A1 | 01-08-2013 | US 2013192595 A1 | 01-08-2013 |
| | | US 2019374739 A1 | 12-12-2019 |
| | | US 2024399098 A1 | 05-12-2024 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 560928 A
- EP 1516639 A
- EP 3821929 A
- EP 3233171 A
- EP 3410927 A
- EP 4209243 A
- EP 4241817 A

- EP 4241812 A
- EP 4295882 A
- US 2022106189 A
- US 11833309 B
- US 2013118486 A
- US 2013192595 A